(19) Europäisches Patentamt
European Patent Office
Office européen des brevets

(11) **EP 2 210 590 A1**

(12) **EUROPEAN PATENT APPLICATION**

(43) Date of publication:
28.07.2010 Bulletin 2010/30

(51) Int Cl.:
*A61K 9/127* (2006.01)          *A61K 31/198* (2006.01)
*A61K 47/48* (2006.01)

(21) Application number: 09151332.5

(22) Date of filing: 26.01.2009

(84) Designated Contracting States:
AT BE BG CH CY CZ DE DK EE ES FI FR GB GR
HR HU IE IS IT LI LT LU LV MC MK MT NL NO PL
PT RO SE SI SK TR
Designated Extension States:
AL BA RS

(71) Applicant: Academisch Medisch Centrum
1100 DE  Amsterdam (NL)

(72) Inventor: Heger, Michal
1076 VX, Amsterdam (NL)

(74) Representative: Van Someren, Petronella F. H. M.
Arnold & Siedsma
Sweelinckplein 1
2517 GK Den Haag (NL)

(54) **Drug delivery system for use in the treatment of vascular and vessel-related pathologies**

(57)      The present invention relates to a drug delivery system for use in the treatment of vascular and vessel-related pathologies, comprising a drug delivery platform that comprises at least one compound capable of exerting an effect on the formation and/or maintenance of a thrombus in the vessel to be treated. The platform is preferably formed by liposomes that are sterically stabilized by grafting of poly(ethylene glycol) onto the liposome surface. The liposomes may further comprise photosensitizers and targeting molecules. The liposomes may be thermosensitive. The compound is suitably tranexamic acid. The drug delivery system is preferably used for the treatment of port wine stains.

Figure 4

Figure 4

ANIONIC LIPOSOMES

CONVENTIONAL LIPOSOMES

STEALTH LIPOSOMES

TARGETED LIPOSOMES

| | |
|---|---|
| Hydrophilic drug | Antibody |
| Hydrophobic drug | Antibody fragment |
| Functionalized photosensitizer | Peptide |
| Hydrophobic anchor molecule | |
| Photocleavable group | |

Printed by Jouve, 75001 PARIS (FR)

**Description**

The present invention relates to a drug delivery system for use in the treatment of vascular and vessel-related pathologies, in particular port wine stains (PWS), by means of selective photothermolysis.

PWS are congenital vascular lesions characterized by ectatic capillaries and post-capillary venules (30-300 $\mu$m in diameter) in the papillary and mid-reticular layers of the dermis. These birthmarks occur in 0.3-0.5% of infants and initially appear as flat, pink maculae that gradually progress into hypertrophic, red-to-purple lesions, typically in proportion to the person's age.

Although the exact etiological origin remains unknown, it has been suggested that progressive hypertrophy of the lesions is caused by low neural densities at the periphery of the ectatic vessels, which accounts for inadequate neurotrophism and tonus regulation of the affected vasculature. An increased perfusion pressure and age-related collagen degeneration in the dermis are possible contributory factors to the vascular hyperdilation. By age 46, two-thirds of the affected individuals develop papular or nodular components resulting from soft tissue overgrowth, causing dysmorphosis, asymmetry, and spontaneous bleeding.

Additionally, the aberrant cosmetic appearance of PWS may significantly impede the individual's psychosocial development and well-being, and constitutes a considerable factor in the overall treatment of PWS, since 70-80% of these birthmarks occur in the head and neck regions.

The anatomical location and dermatomal distribution pattern of trigeminal PWS (pertaining to the ophthalmic, maxillary, and mandibular branches of the trigeminal nerve located in the respective regions of the face) have been linked to a heightened probability of ocular and/or central nervous system complications (glaucoma and Sturge-Weber syndrome, respectively). Other PWS-related disorders have been identified, further underscoring the need for an effective therapeutic modality.

Photocoagulation is based on the selective destruction of blood vessels by laser irradiation as the result of a photothermal response. When blood vessels are irradiated at a wavelength preferentially absorbed by hemoglobin (typically 580-600 nm), the radiant energy is converted to heat that subsequently diffuses from the so-called nucleation centers (red blood cells) to lower thermal regions. The generation and diffusion of supracritical temperatures (>70°C) induces thermal denaturation of blood and, depending on the extent of diffusion and convection, the vascular wall and perivascular tissue.

Because the wavelengths used for photocoagulation are not well-absorbed by perivascular tissue constituents, non-vascular tissue remains spared when the laser pulse duration is kept within the thermal relaxation time of the target vessels, defined as the time required for a tissue volume to lose 50% of its thermal energy through diffusion and convection. At appropriate pulse durations, normal-sized capillaries (4-6 $\mu$m inner diameter) and post-capillary venules (8-26 $\mu$m inner diameter), which have a relatively short thermal relaxation time and a smaller thermal mass, therefore remain spared during longer pulse durations, as heat diffusion from these vessels precludes the generation of denaturing temperatures.

The generation of supracritical temperatures inside the vascular lumen leads to the denaturation of blood, which in turn results in the formation of a thermal coagulum: an amorphous clump of denatured material (plasma proteins, blood cells, etc.) that has formed in supracritically heated regions. The formation of thermal coagula in laser-irradiated blood vessels has been demonstrated in humans by histological analysis of laser-treated port wine stains [Hohenleutner U et al. J Invest Dermatol. 1995 May;104(5):798-802., Fiskerstrand EJ et al. J Invest Dermatol. 1996 Nov;107(5):671-5] and in animal models [Heger M et al. Opt Express. 2005 Feb;13(3):702-15, Suthamjariya K et al. J Invest Dermatol. 2004 Feb;122(2):518-25, Bezemer R et al. Opt Express. 2007 Jul;15(14):8493-506].

The efficacy of selective photothermolysis depends on a combination of inevitable intrinsic factors: epidermal pigmentation, optical shielding by blood and superimposed vessels, and PWS anatomy and morphology. Generally, treatment efficacy correlates negatively with increased melanin content, vascular density and superimposition, and vessel diameter and depth, provided that the prominence of these factors is inversely proportional to the optical penetration depth. Consequently, incomplete photocoagulation may result from the generation of subcritical isotherms as a result of inhomogeneous photon distribution in the lumen (as is the case with large diameter vessels), or may be forestalled altogether by insufficient heat production across the entire vessel diameter (such as in deeply situated or optically shadowed vessels). The intraluminal gyrations in fluence rates ($J/cm^2$) have a profound effect on the acute tissular and hemodynamic responses, and ultimately lesional clearance, which occurs through inflammation-mediated reduction in dermal blood volume.

The laser-induced production of supracritical temperatures within the entire luminal volume leads to widespread thermal necrosis of the vessel wall and vaso-occlusion by the thermolysed and agglutinated chromophore-containing red blood cells. Clinically, complete photocoagulation of the vascular lumen is associated with well-responding lesions [Fiskerstrand EJ et al. J Invest Dermatol. 1996 Nov;107(5):671-5], corresponding to approximately 40% of the cases [Greve B et al. Lasers Surg Med. 2004;34(2):168-73]. In contrast, moderately responding (20-46%) and refractory (14-40%) PWS have a post-treatment vascular profile characterized by varying degrees of partially photocoagulated vessels with semi-obstructive thermal coagula [Black JF et al. Photochem Photobiol. 2004 Jul-Aug;80:89-97, Tan OT et al. Arch Dermatol. 1986 Sep;122(9):1015-22].

Inasmuch as the existing laser therapy is not effective in approximately 60% of the cases, it is the object of the present invention to provide a means to improve the clearance rate.

The invention thus provides an adjuvant modality to be used in conjunction with conventional photocoagulation (by selective photothermolysis) which improves lesional clearance rates by optimizing the occlusion of target blood vessels.

In the research that led to the invention it was shown that, in PWS vascular analogues (hamster dorsal skin fold venules), the photothermal response is ensued by a hemodynamic response, namely the initiation of primary and secondary hemostasis following laser-induced endovascular damage. There is increasing evidence that misfolded proteins and corollary fibrillar structures referred to as amyloid have the propensity to activate platelets (Herczenik E et al. Arterioscler Thromb Vasc Biol. 2007 Jul;27(7):1657-65) and the contact activation pathway (Maas C et al. J Clin Invest. 2008 Sep;118(9):3208-18). Inasmuch as thermal coagula are comprised of thermally denatured (i.e., misfolded) proteins, these laser-induced lesions may constitute the basis for the initiation of primary and secondary hemostasis in addition to a thermally afflicted endothelium.

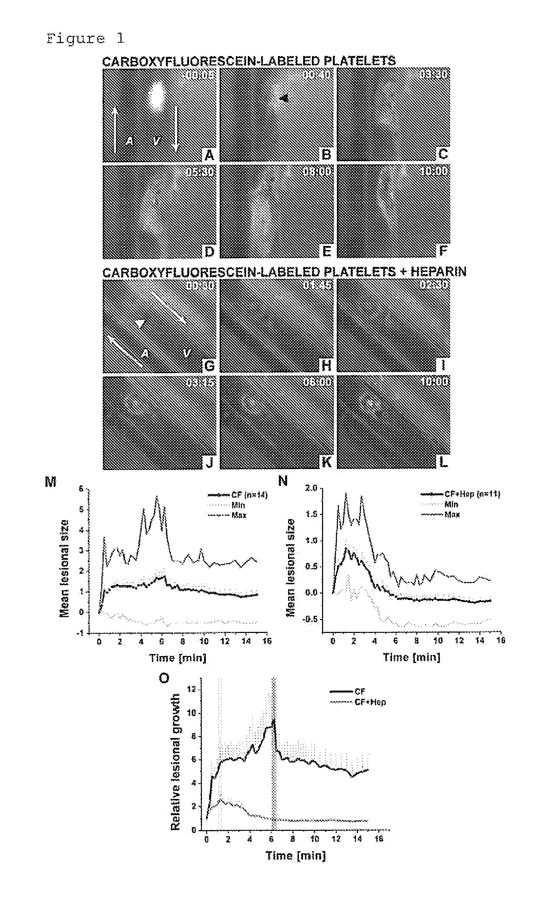

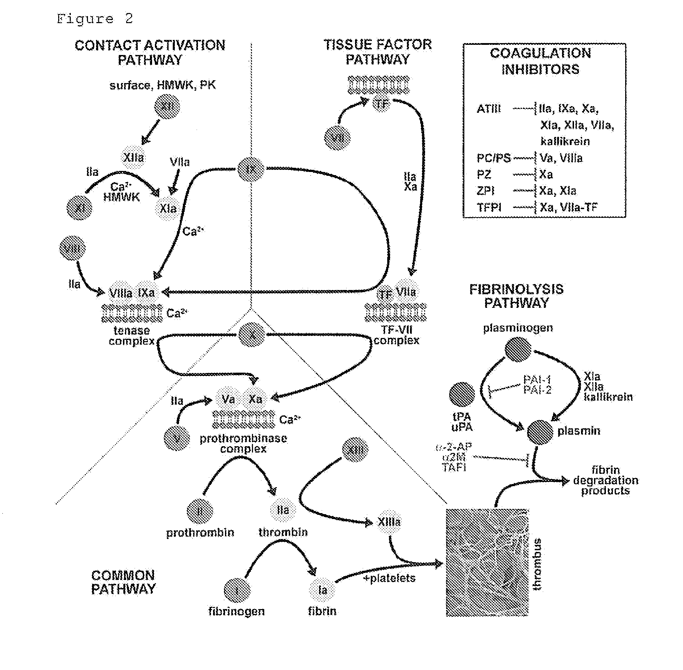

The primary hemostatic response is characterized by platelet aggregation around the laser-induced lesion (in cases where the thermal coagulum remains attached to the vessel wall) or at the vascular wall where the thermal coagulum was induced (in cases where the thermal coagulum dislodged following the laser pulse) [Bezemer R et al. Opt Express. 2007 Jul;15(14):8493-506]. We have demonstrated that 5,6-carboxyfluorescein-labeled platelets accumulate on the thermal coagulum and at the laser-irradiated vascular wall (**Figure 1A-F,M,O**) and that thrombus formation peaks at 6.15 min, marking the subsequent onset of fibrinolysis. This process is partially inhibited by the infusion of anti-glycoprotein Iba antibodies, indicating that platelets (primary hemostasis) are implicated in the hemodynamic response. Additionally, infusion of heparin exhibited an impeding effect on the lesional size (**Figure 1G-L,N,O**), indicating that the coagulation cascade (secondary hemostasis) **(Figure 2)** also plays a role in laser-induced thrombosis.

It was further shown that prothrombotic and/or antifibrinolytic pharmaceutical agents have the ability to enhance endoluminal emphraxis via amplified thrombus formation and preserved thrombus integrity, respectively, in semi-photocoagulated vasculature, which will result in optimized lesional clearance rates through the consequent chronic inflammatory responses and corollary vascular remodeling. The therapeutic efficacy of selective photothermolysis of PWS and other vascular and vessel-related pathologies can thus be enhanced by the administration of prothrombotic and/or antifibrinolytic pharmaceutical agents prior to selective photothermolysis.

According to a first aspect thereof, the invention relates to the use of a compound capable of exerting an effect on the formation and/or maintenance of a thrombus for the treatment of vascular and vessel-related pathologies, in particular PWS, by selective photothermolysis. Such compounds are prothrombotic and/or antifibrinolytic pharmaceutical agents.

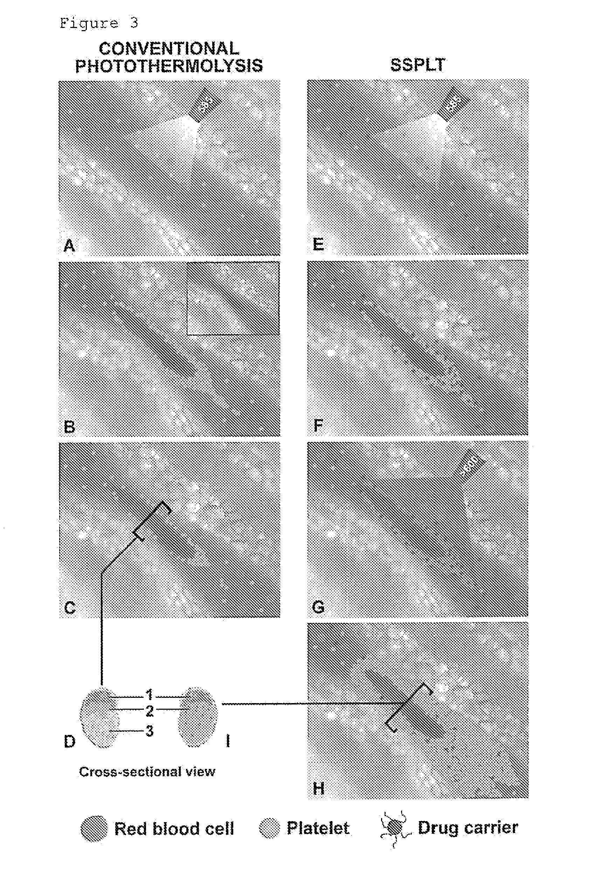

The potential hazard of parentally administering prothrombotic and/or antifibrinolytic substances to non-coagulopathic patients is impairment of the hemostatic "checks and balance" system. Consequently, the pharmaceutical efficacy should preferably be constrained to the region to be treated only, insofar as regulation of naturally occurring hemostatic events is not compromised. In order to achieve this, the invention provides a drug delivery system (DDS) for use in the treatment of vascular and vessel-related pathologies, comprising a drug delivery platform that comprises at least one compound capable of exerting an effect on the formation and/or maintenance of a thrombus in the vessel to be treated. The combined therapeutic modality, i.e., selective photothermolysis in conjunction with the use of a pharmaceutical agent-encapsulating DDS, is referred to as site-specific pharmaco-laser therapy (SSPLT). The principal components of SSPLT are depicted in **Figure 3.**

A DDS of the invention for SSPLT preferably possesses the following attributes: stable physicochemical properties with minimal passive release of the encapsulated drug over time, high encapsulation efficiency since enclosure of the drug will limit its bioavailability, targeting capacity to the site of laser-induced damage, an efficacious drug release mechanism, and low immunogenicity.

## *LIPOSOME COMPOSITION*

The DDS can be any of the existing platforms, including liposomes, polymeric drug carriers, cells, and cell ghosts. Liposomes, however, constitute the most advantageous carrier system due to the facile preparation techniques (that allow bulk production), their manipulatable attributes, and their ability to encapsulate hydrophilic and lipophilic molecules at high efficiencies. In addition to the inherent non-toxicity of neutral phospholipids, liposomes can be modified compositionally to facilitate the unique prerequisites of the DDS.

Preferably, the head group of the lipids comprising the liposomal DDS is selected from the group consisting of: phosphocholine, phosphoethanolamine, phosphatidic acid, phosphoglycerol, phosphoserine, phosphoinositol, sphingosine, diglycerophosphate, glycerol, ethylene glycol, galloylglycerol, and glycero-3-succinate.

The acyl chain of the lipid is preferably selected from the group consisting of: tridecanoyl (13 carbons), myristoyl (14 carbons), myristoleoyl (14 carbons, cis-alkene at $\Delta_9$), myristelaidoyl (14 carbons, trans-alkene at $\Delta_9$), pentadecanoyl (15 carbons), palmitoyl (16 carbons), palmitoleoyl (16 carbons, cis-alkene at $\Delta_9$), palmitelaidoyl (16 carbons, trans-alkene at $\Delta_9$), phytanoyl (16 carbons, methylated at $\Delta_{3,7,11,15}$), heptadecanoyl (17 carbons), stearoyl (18 carbons), petroselinoyl (18 carbons, cis-alkene at $\Delta_6$), oleoyl (18 carbons, cis-alkene at $\Delta_9$), elaidoyl (18 carbons, trans-alkene at $\Delta_9$), linoleoyl (18 carbons, cis-alkenes at $\Delta_{9,12}$), linolenoyl (18 carbons, cis-alkenes at $\Delta_{9,12,15}$), nonadecanoyl (19 carbons), arachidoyl (20 carbons), eicosenoyl (20 carbons, cis-alkene at $\Delta_{11}$), arachidonoyl (20 carbons, cis-alkenes at $\Delta_{5,8,11,14}$), heniecosanoyl (21 carbons), behenoyl (22 carbons), erucoyl (22 carbons, cis-alkene at $\Delta_{13}$), docosahexaenoyl (22 carbons, cis-alkenes at $\Delta_{4,7,10,13,16,19}$), trucisanoyl (23 carbons), lignoceroyl (24 carbons), nervonoyl (24 carbons, cis-alkene at $\Delta_{15}$).

In a preferred embodiment of liposomes of the invention the lipids have a monoacyl (1-acyl-2-hydroxy-*sn*-glycero-3-head group) or diacyl (1-acyl-2-acyl-*sn*-glycero-3-head group) configuration.

Enhancement of the in vivo circulation time can be accomplished by proper sizing. The methods employed for sizing are well known in the art and for example described in Awasthi VD et al. Int J Pharm. 2003 Mar 6;253(1-2):121-32. Suitable liposomes for use as the drug delivery platform in the drug delivery system of the invention have a size between 30 and 1500 nm, preferably between 90 and 200 nm [Liu D et al. Biochim Biophys Acta. 1992 Feb 17:1104(1):95-101], more preferably between 160 and 200 nm, and most preferably about 180 nm.

### *STERIC STABILIZATION*

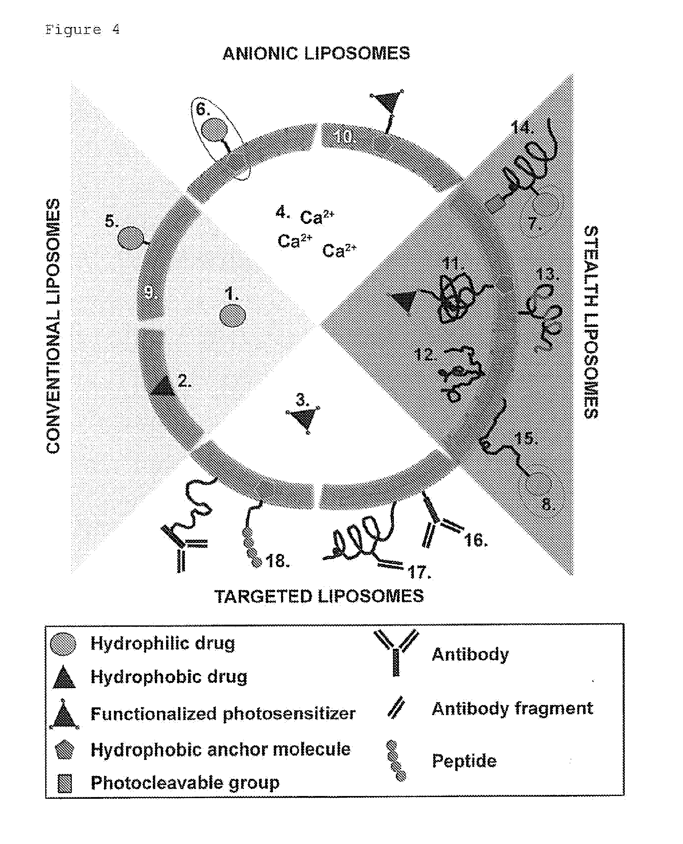

In order to prevent liposome aggregation and fusion and to enhance circulatory half life, the liposomes are preferably sterically stabilized. Methods for sterical stabilization are for example described in [Klibanov AL et al. FEBS Lett. 1990 Jul 30;268(1):235-7, Senior J et al. Biochim Biophys Acta. 1991 Feb 11;1062(1):77-82, Allen TM et al. Biochim Biophys Acta. 1991 Jul 1;1066(1):29-36]. In a preferred embodiment this is achieved by the grafting of poly(ethylene glycol) (PEG, also referred to as polyethylene oxide (PEO) or polyoxyethylene (POE)) onto the liposomal surface as described in [Klibanov AL et al. FEBS Lett. 1990 Jul 30;268(1):235-7, Allen TM et al. Biochim Biophys Acta. 1991 Jul 1;1066(1):29-36, Blume G et al. Biochim Biophys Acta. 1990 Nov 2;1029(1):91-7]. This can be effected by including a molar fraction of linear or branched PEG [Torchilin VP et al. J Pharm Sci. 1995 Sep;84(9):1049-53] covalently linked to a lipid constituent (usually phosphatidylethanolamine (PE) or phosphatidylglycerol (PG)) or to a hydrophobic anchor molecule in the lipid bilayer, such as, but not limited to, cholesterol, poly(propylene oxide) (PPO), or mono- or diacyls **(Figure 4)**. The presence of a "dense conformational cloud" by the PEG polymers over the liposome surface, the repulsive interactions between PEG-grafted membranes and blood constituents, the hydrophilicity of PEGylated formulations, and the decreased rate of plasma protein adsorption on the hydrophilic surface of PEGylated liposomes impose so-called 'stealth' properties through which rapid clearance by cells of the reticuloendothelial system is considerably forestalled. The use of these techniques for 'stealthing' is part of the present invention.

In further embodiments, the liposomes are long circulating. Enabling the liposomes to be long circulating can be achieved by various techniques. One example is by the inclusion of covalently linked polymers, diblock copolymers, and/or multiblock copolymers selected from the group of poly(vinyl alcohol) (PVA), polyglycerols, poly(N-vinylpyrrolidone) (PVP) that is activated as succinimidyl ester and bound to the amine-containing anchor (usually PE), poly(N-acryloyl) morpholine (PAcM) that is activated as succinimidyl ester and bound to the amine-containing anchor (usually PE), poly (2-ethyl-2-oxazoline) (PEOZ), poly(2-methyl-2-oxazoline) (PMOZ), polyacrylamide, poly(N-isopropylacrylamide) (NIPAM), poly[N-(2-hydroxypropyl)methacrylamide] (HPMA), poly(styrene-co-maleic acid/anhydride) (SMA), poly(divinyl ether maleic anhydride) (DIVEMA), and/or hydrophobized polysaccharides selected from the group of pullulan, dextran, mannan, and/or polysialic acids, and/or glucuronic acids selected from the group of palmitylglucuronide (PG1cUA), palmitylgalacturoide, and/or gangliosides and sialic acid derivatives selected from the group of monosialoganglioside (GM1), GM3.

In a further embodiment, anionic liposomes, i.e., liposomes in part composed of anionic constituents, are long circulating by the (electro-attractive) adsorption of polymers, diblock copolymers, and/or multiblock copolymers consisting of cationic residues selected from the group of quaternized poly(4-vinylpyridine) (PEVP), poly(ethyleneimine) (PEI), polybetaines (PB).

### *STERIC STABILIZATION BY DESORBABLE/PHOTOCLEAVABLE PEG*

As presented below, several embodiments pertaining to liposomal formulations for which direct contact between plasma components and the liposomal surface is exacted ultimately may not benefit from the grafting of low-immunogenic polymers to the liposomal surface. Steric stabilization may impede the accessibility of the target plasma components to the liposomal membrane constituents. Converesely, the systemic administration of sterically unstabilized drug carriers, and especially those with a negative zeta potential, causes these carriers to be opsonized at a substantially greater rate.

Sterically unstabilized drug carriers that contain grafted peptides, antibodies, or antibody fragments on the outer surface are also more subject to uptake by the reticuloendothelial system.

In order to circumvent this dichotomy, the DDS of choice may be stabilized with desorbable or cleavable steric stabilizers for the specific purpose of exposing DDS-incorporated or bound constituents that mediate an antifibrinolytic or prothrombotic response as described in the invention. In a first preferred embodiment, this is achieved by incorporation of desorbable PEG-derivatized PE such as PEG-PE of varying acyl chain lengths into anionic liposomes, as has been described for phosphatidylserine (PS) liposomes in [Chiu GN et al. Biochim Biophys Acta. 2002 Feb 18;1560(1-2) : 37-50] and [Chiu GN et al. Biochim Biophys Acta. 2003 Jun 27;1613(1-2):115-21].

In another preferred embodiment, the DDS comprises liposomes containing PEG-modified plasmenyl-type lipids. Plasmenyl-type lipids, or plasmalogens, are ether lipids that contain a linear acyl chain connected to the glycerol backbone at the *sn*-1 (plasmalogens) and *sn*-2 position (diplasmalogens) via a vinyl residue (from a vinyl alcohol) and an alkene at $\Delta_1$ instead of the typical ester, and usually a phosphocholine or phosphoethanolamine attached to the *sn*-3 carbon of glycerol. Plasmalogens are predominantly located in the heart (~50% of PE contains the alkenyl ether) and protect cells against the damaging effects of singlet oxygen ($^1O_2$) and reactive oxygen species (ROS). $^1O_2$ and ROS attack the plasmalogen at the electrophilic vinyl ether linkage to form a single-chain surfactant (with a fatty aldehyde and a dioxymethyl as byproducts) that induces lamellar-to-hexagonal phase changes (type-I micellar structure for plasmenylcholine and a type-II micellar structure for plasmenylethanolamine).

Inasmuch as these vinyl ethers are very susceptible to acidic and oxidative conditions, they can be employed in a broad range of DDS-related applications when such conditions are present or induced. One possible application relates to the photo-oxidative removal of PEG from PEG-derivatized diplasmalogen-containing liposomes. In this specific embodiment, liposomes containing thrombogenic phospholipids and liposomes with grafted prothrombotic and/or antifibrinolytic compounds on the surface as enabled in this invention are protected from opsonization through the incorporation of a molar fraction of PEG-derivatized plasmalogens. The synthesis routes of these PEG-plasmalogens are known and have for example been described in [Thompson DH et al. Methods Enzymol. 2004;387:153-68]. The presence of PEG on the outer surface serves to protect the biologically active compounds from interacting with their respective plasma/cellular targets. The deprotection by dePEGylation, and thus activation of the DDS, is achieved by the generation of $^1O_2$ and ROS by means of a photosensitizer built into the DDS as described in this invention and the consequent cleavage of the vinyl ether and release of PEG.

The dePEGylation modality **(Figure 4)** can be very suitably employed in combination with the photosensitizer-based procoagulant SSPLT as described below.

## *METHODS OF ENCAPSULATION/GRAFTING*

In a preferred embodiment, the pharmaceutically active compound capable of exerting an effect on the formation and/or maintenance of a thrombus is either encapsulated in the aqueous compartment or the phospholipid bilayer of the liposome. In cases when multiple compounds are encapsulated, the compounds may be present in both the aqueous compartment of the liposome as well as the bilayer or linked to a DDS constituent. Various compounds can be present in various compartments **(Figure 4)**.

A number of molecular components of primary hemostasis, secondary hemostasis, and the fibrinolytic cascade can only be targeted with compounds that are not suitable for liposomal encapsulation (e.g., (short, oligo-, poly-) peptides, proteins (recombinant, modified, or purified), and antibodies or Fab fragments because they are either heat-labile (in case of thermosensitive liposomes) and/or too large for transmembrane passage. In an alternative embodiment, such compounds may be (covalently) attached to a component phospholipid (such as 1,2-diacyl-*sn*-glycero-3-phosphoethanolamine) , an anchor molecule embedded in the bilayer, or a polymer used for steric stabilization of the liposomes, such as PEG, whereby the polymer may contain side chains or R-groups for linking the pharmaceutically active compound **(Figure 4)**.

In a further alternative embodiment, such compounds may be co-infused into the systemic circulation in unencapsulated form before or directly after laser therapy. This applies for example to antifibrinolytic drugs such as tranexamic acid (TA), e-aminocaproic acid (ACA), *p*-aminomethylbenzoic acid (AMBA), and 4-aminomethyl-bicyclo-2,2,2-octane carboxylic acid (AMBOCA) because these drugs do not exert an effect until the manifestation of a thrombotic event. Consequently, these drugs pose a reduced risk for disturbing the hemostatic equilibrium, particularly when administered at subclinical, adjuvant dosages.

## *ANTIFIBRINOLYTICS*

The compound capable of exerting an effect on the formation and/or maintenance of a thrombus in the vessel to be treated may exert this effect at the level of any of the components of the fibrinolytic pathway, the tissue factor or contact activation pathways (secondary hemostasis), or platelet function (primary hemostasis).

The fibrinolytic pathway involves the conversion of plasminogen into plasmin, which cleaves cross-polymerized fibrin into soluble fibrin degradation products, thereby dissociating the thrombus. Fibrin degradation products in turn compete with thrombin, and so retard the conversion of fibrinogen to fibrin. A schematic overview of fibrinolysis is presented in **Figure 2.** According to the invention, fibrinolysis of the thrombus that has formed as a result of photocoagulation is to be deterred. The inhibition of fibrinolysis by pharmaceutical intervention will preserve thrombus integrity and promote thrombus stability during and after laser-induced thrombus formation, delaying or forestalling the onset of gradual thrombus dissolution as a result of fibrinolysis and shear stress.

Suitable fibrinolysis-targeting compounds to be encapsulated in the DDS of choice, and in particular in liposomes, are selected from the group consisting of inhibitors of one or more components of the fibrinolytic system that promote the formation of plasmin or fibrin degradation products or components of the fibrinolytic system, or their agonists, that deter the formation of plasmin or fibrin degradation products.

In a first embodiment, the compound is an inhibitor of plasmin(ogen). Plasmin, which is gradually formed at the onset of blood coagulation, is responsible for cleaving cross-polymerized fibrin strands that make up the reticular network of the thrombus. The plasmin(ogen) inhibitor is selected from the group of fatty acids comprising arachidonate, oleate, stearate (which can be incorporated into the lipid bilayer of the liposomal DDS) and preferably from synthetic plasmin(ogen) inhibitors comprising TA, ACA, AMBA, AMBOCA, more preferably the inhibitor is TA or ACA, and most preferably the inhibitor is TA. All synthetic inhibitors of plasmin(ogen) mentioned are zwitterionic at neutral pH (pH = 7.4) and are encapsulated in the aqueous compartment of the liposomes.

TA is an antifibrinolytic lysine analogue that is widely used in the clinical setting to deter peri- and postoperative blood loss in cardiac surgery and other highly invasive procedures. TA is also prescribed as a prophylactic for patients with hemophilia and von Willebrand disease, as well as for excessively mennorhagic women. TA completely antagonizes the biological activity of plasmin(ogen) by occupying its five lysine-binding sites, thereby inhibiting the formation of a molecular complex required for fibrinolysis. The pharmacokinetics of TA have been extensively studied and TA is considered safe at the prescribed dosimetries [U.S. Food and Drug Administration approval of application # 019280 (supplement # 008) and application # 019281 (supplement # 009), approval date 9/9/1999]. TA constitutes the preferred compound for inclusion in the drug delivery system of the invention.

In a first alternative embodiment, the compound is an inhibitor of plasmin. Examples of compounds that are direct inhibitors of plasmin include $a_2$-antiplasmin, $a_2$-macroglobulin, and thrombin-activatable fibrinolysis inhibitor (TAFI). Inhibitors of plasmin are selected from the group of purified and/or recombinant $a_2$-antiplasmin, $a_2$-antiplasmin polypeptides, $a_2$-macroglobulin, purified and/or recombinant TAFI, and/or aprotinin.

In a second alternative embodiment, the compound is an inhibitor of tissue plasminogen activator (tPA) or urokinase-type plasminogen activator (uPA). tPA and uPA, which are secreted into the blood by damaged and activated endothelium, mediate fibrinolysis by converting thrombus-trapped plasminogen to plasmin. A positive feedback loop propagates the fibrinolytic state in that plasmin further stimulates plasmin generation by producing more active forms of both tPA and uPA. tPA and uPA are inhibited by plasminogen activator inhibitor-1 (PAI1) and plasminogen activator inhibitor-2 (PAI-2). It is preferred that tPA inhibitors are selected from the group of isolated and purified human PAI1, isolated and purified human PAI2, recombinant human PAI1, recombinant human PAI2, modified human PAI1, modified human PAI2, an inhibitory antibody, or a derivative thereof (e.g., a Fab fragment), directed against tPA, a (poly-, oligo-, or short) peptide that inhibits tPA (cf. US-6,159,938).

It is preferred that uPA inhibitors are selected from the group of isolated and purified human PAI1, isolated and purified human PAI2, recombinant human PAI1, recombinant human PAI2, modified human PAI1, modified human PAI2, an inhibitory antibody, or a derivative thereof (e.g., a Fab fragment), directed against uPA, an inhibitory antibody, or a derivative thereof (e.g., a Fab fragment), directed against the uPA receptor (uPAR), a (poly-, oligo-, or short) peptide that inhibits uPA (cf. US-6,159,938).

In a further alternative embodiment, the compound is an agonist of PAI1 or PAI2, i.e., an agent that induces the secretion of PAI1 or PAI2. For PAI1 such agonist is in particular a synthetic peptide derived from the fragment $S^{362}$-$A^{380}$ of vitronectin, referred to as BP4, or a synthetic peptide such as SFLLRN that promotes PAI1 secretion by binding to proteinase-activated receptor-1 (PAR-1). For PAI2 such agonist is in particular a synthetic peptides that promotes PAI2 secretion, e.g. SLIGKV, which binds to proteinase-activated receptor-2 (PAR2), or synthetic peptides that prevent PAI2 polymerization under physiological conditions, e.g., TEAAAGTGGVMTG (RCL-PAI2) and SEAAASTAWIAG (RCL-AT), which may impair PAI2 functionality in the circulation [Mikus P, Ny T. Intracellular polymerization of the serpin plasminogen activator inhibitor type 2. J Biol Chem. 1996 Apr 26;271(17):10048-53].

### *PROCOAGULANTS - TISSUE FACTOR PATHWAY AND CONTACT ACTIVATION PATHWAY*

In addition to or instead of the antifibrinolytic component of the DDS, an agent may be encapsulated that induces a procoagulant response by acting on one or more components of secondary hemostasis, namely the tissue factor and contact activation pathways **(Figure 2)**. The coagulation system encompasses a complex cascade of clotting factors (e.g., factor VII, or fVII) that are converted to their active form (e.g., fVIIa), whereby an activated clotting factor in turn activates the next zymogen in the cascade. Both the tissue factor and contact activation pathways lead to the conversion of fibrinogen (fI) to fibrin (fIa), which polymerizes and fortifies the clot by cross-linking platelets and by forming a reticular network throughout the thrombus.

A procoagulant state can be induced by compounds that mediate secondary hemostasis or antagonists of one or more inhibitors of components of secondary hemostasis.

Examples of mediators of the tissue factor and contact activation pathway include fII(a) in purified form, recombinant form, or as part of a commercially available pharmaceutical preparation; fIII (tissue factor, TF), fV(a) in purified form or recombinant form; fVII in purified form, recombinant form, or as part of a commercially available pharmaceutical preparation; fVIII(a) in purified form or recombinant form; fTX(a) in purified form, recombinant form, or as part of a commercially available pharmaceutical preparation; fX(a) in purified form, recombinant form, or as part of a commercially available pharmaceutical preparation; fXI(a) in purified form or recombinant form; fXII in purified form or recombinant form; fXIII(a) in purified form or recombinant form, prekallikrein (PK), kallikrein, high-molecular-weight kininogen (HMWK).

## PROCOAGULANTS - ANTAGONISTS OF COAGULATION INHIBITORS

In order to proportionate the extent of coagulation to the extent of vascular damage, a number of coagulation factor inhibitors exert an anticoagulant effect during thrombosis. The most prominent inhibitor is antithrombin III (ATIII), a plasma-borne glycoprotein that inhibits proteases of both the contact activation (fIXa, FXa, fXIa, fXIIa) and tissue factor (fVIIa) pathway, and fIIa produced in the common pathway. ATIII further inhibits kallikrein. A second important component is protein C, a vitamin K-dependent serine protease enzyme that is activated by thrombin-bound thrombomodulin on the endothelial cell outer membrane surface to form activated protein C (APC) in the presence of cofactor protein S. APC is responsible for the degradation of fVa and fVIIIa. The third major anticoagulant is tissue factor pathway inhibitor (TFPI), a single-chain polypeptide that reversibly inhibits fXa, and, when complexed to fXa, can subsequently also inhibit the fVIIa-TF complex. Finally, protein Z-dependent protease inhibitor (ZPI) is a blood-borne serpin (serine protease inhibitor) that inhibits fXIa and fXa. The latter occurs in conjunction with protein Z, a glycoprotein that accelerates the degradation of fXa by ~1000-fold.

In a further embodiment, the DDS of choice may encapsulate or have engrafted antagonists of coagulation inhibitors selected from the group of ATIII, protein C, protein S, APC, thrombomodulin, TFPI, ZPI, protein Z. Antagonists of coagulation inhibitors are selected from the group of (short, oligo-, poly-) peptides, proteins (recombinant, modified, or purified), and antibodies or Fab fragments. The antagonists may be encapsulated in the DDS or grafted onto a component phospholipid, anchor molecule, or a polymer chain used for steric stabilization as decribed above.

The procoagulant agents and antagonists of anticoagulants (e.g., antibodies) that are currently available are sometimes less suitable for encapsulation into (thermosensitive) liposomes because these compounds are heat-labile proteins that may have impaired membrane permeability due to their large size. Moreover, these classes of drugs require elaborate GMP-controlled preparation and processing, which often translates into high product pricing.

## PROCOAGULANTS - PHOTOSENSITIZERS

In a further embodiment the invention provides an alternative approach to circumvent the use of 'classical' procoagulants. This approach is based on the inclusion of a photosensitizer into the hydrophobic core of the lipid bilayer or the aqueous compartment of the liposomes (in which case the photosensitizer is functionalized with hydrophilic moieties).

Photosensitizers are a class of molecules that, when brought to an excited electronic state through the input of energy (e.g., light), transfer a portion of the energy to neighbouring molecules, typically molecular oxygen, during electron decay to the ground state. Photosensitizers are used primarily in photodynamic therapy (PDT), in which they act as electron donors and facilitate the formation of highly cytotoxic and thrombogenic singlet oxygen ($^1O_2$) and reactive oxygen species (ROS). The generation of these reactive transients during PDT results in irreversible tissue destruction, conferring a selective therapeutic effect to a volume of tissue containing the photosensitizer. In the case of the procoagulant DDS of choice, reactive oxygen species are formed that may damage the cells that comprise the thrombus and further damage the vessel wall, thus leading to additional thrombus formation.

In a preferred embodiment of the invention, a photosensitizer is encapsulated in a separate liposomal formulation to comprise a DDS with procoagulant properties.

Suitable photosensitizers for use in the invention are selected from the group of phthalocyanines, naphthalocyanines, and porphins selected from the group of chlorins and bacteriochlorins.

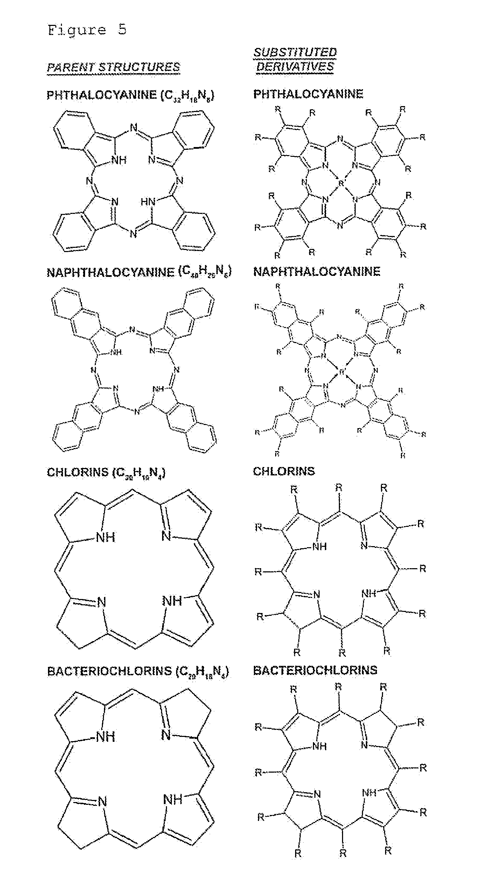

Photosensitizers that are particularly useful in the invention are molecules of the general formulas $C_{32}H_{18}N_8$ (phthalocyanines), $C_{48}H_{26}N_8$ (naphthalocyanines), $C_{20}H_{16}N_4$ (chlorins) or $C_{20}H_{18}N_4$ (bacteriochlorins) as illustrated in **Figure 5,** which are optionally substituted with one or more R-groups selected from the group of H, F, $CF(CF_3)_2$, $O(CH_2)$

$n$CF$_3$, Cl, Br, CHCH$_2$, (CH$_2$)$n$CH$_3$, (CH$_2$)$n$COOH, CONH(CH$_2$)$n$NH$_2$, (CH$_2$)$n$CONHCH$_2$CH$_2$NH$_2$, CONH(CH$_2$)$n$CH(NH$_2$) COOH, CH$_2$CONHCH(CH$_2$COOH)COOH, O(CH$_2$)$n$CH$_3$, S(CH$_2$)$n$N(CH$_3$)$_2$, SO$_2$NH(CH$_2$)$n$CH$_3$, SO$_2$NH(CH$_2$)$n$N(CH$_3$)$_2$, SO$_2$N[(CH$_2$)$n$CH$_3$]$_2$, SO$_2$NHCH$_2$CH(CH$_2$CH$_3$)(CH$_2$)$n$CH$_3$, C(CH$_3$)$_3$, OC[(CH$_2$)$n$CH$_3$]$_3$, OCH[CH(CH$_3$)$_2$]$_2$, O(CH$_2$)$n$N (CH$_3$)$_2$, O(CH2)$n$N(CH$_3$)$_3$, SC$_6$H$_5$, OC$_6$H$_5$, O(C$_6$H$_4$)C[(CH$_3$)$_2$](C$_6$H$_5$), O(C$_6$H$_3$)(COOH)$_2$, O(C$_6$H$_3$)[COO(CH$_2$)nCH$_3$]$_2$, C$_6$H$_5$, SO$_2$NHCH(CH$_3$)CH$_2$(C$_6$H$_3$)(OCH$_3$)$_2$, SO$_3$, SO$_2$Cl, N(CH$_3$)$_2$, COOH, NO$_2$, CH$_3$, CONH$_2$, CH$_2$NH$_2$, and the R'-group is selected from the group of H, CH$_3$, AlCl, AlOH, AlOSi(CH$_3$)$_3$, AlOSO$_3$, Co, Cu, Li, GaOH, GaCl, Fe, FeCl, FeO$_2$, Pb, Mg, Mn, MnCl, SiCH3Cl, Si(OH)2, Si(Cl)2, Si{OC[(CH$_2$)nCH$_3$]$_3$}$_2$, Si[COCO(C$_6$H$_4$)(CH$_2$)nCH$_3$]$_2$, Si[OSi(CH$_3$)$_2$(CH$_2$) nN(CH$_3$)$_2$]OH, Si[O(CH$_2$)nOCH$_3$]$_2$,Si[OSi(CH$_3$)$_2$(CH$_2$)nN(CH$_3$)$_2$, Si[OSi(CH$_2$)nCH$_3$]$_2$, SiCH$_3$, Si[OSi(CH$_3$)$_2$C(CH$_3$)$_2$C (CH$_3$)$_2$]$_2$, Ni, SnO, Sn(Cl)$_2$, Ti(Cl)$_2$, TiO, VO, Zn, Ag, Cd, Ge, InCl.

Suitable examples of phthalocyanines are 29H,31H-phthalocyanine; 2,9,16,23-tetra-tert-butyl-29$H$,31$H$-phthalocyanine; 1,8,15,22-tetrakis(phenylthio)-29$H$,31$H$-phthalocyanine; 2,9,16,23-tetrakis(phenylthio)-29$H$,31$H$-phthalocyanine; 2,9,16,23-tetraphenoxy-29$H$,31$H$-phthalocyanine; 1,4,8,11,15,18,22,25-octabutoxy-29$H$,31$H$-phthalocyanine; 2,3,9,10,16,17,23,24-octakis(octyloxy)-29H,31H-phthalocyanine; tetrakis(4-cumylphenoxy)-phthalocyanine; 29$H$,31$H$,1,4,8,11,15,18,22,25-octafluoro-2,3,9,10,16,17,23,24-octakisperfluoro(isopropyl)-phthalocyanine; 29H,31H,1,2,3,4,8,9,10,11,15,16,17,18,22,23,24,25-hexadeca-(2,2,2-trifluoroethoxy)-phthalocyanine; zinc phthalocyanine; zinc tetranitrophthalocyanine; zinc 2,9,16,23-tetra-tert-butyl-29$H$,31$H$-phthalocyanine; zinc 2,9,16,23-tetrakis(phenylthio)-29$H$,31$H$-phthalocyanine; zinc 1,4,8,11,15,18,22,25-octabutoxy-29$H$,31$H$-phthalocyanine; zinc 2,3,9,10,16,17, 23,24-octakis(octyloxy)-29$H$,31$H$-phthalocyanine; zinc 1,2,3,4,8,9,10,11,15,16,17,18,22,23,24,25-hexadecafluoro-29H,31H-phthalocyanine; zinc 1,4,8,11,15,18,22,25-octafluoro-2,3,9,10,16,17,23,24-octakisperfluoro(isopropyl)-phthalocyanine; zinc 1,2,3,4,8,9,10,11,15,16,17,18,22,23,24,25-hexadeca-(2,2,2-trifluoroethoxy)-phthalocyanine; zinc 2,3,9,10,16,17,23, 24-octa[(3,5-bispentyloxycarbonyl)phenoxy]-phthalocyanine; zinc 2,3,9,10,16,17,23,24-octa[(3,5-biscarboxylate)-phenoxy]-phthalocyanine; zinc tetrakis(2,4-dimetil-3-pentyloxi)-phthalocyanine; zinc tetrakis(N,N,N-trimethylammoniumetoxi)-phthalocyanine; zinc 1,2,3,4,8,9,10,11,15,16,17,18,22,23,24,25-hexadecachloro-29$H$,31$H$-phthalocyanine; zinc 2,3,9,10,16,17,23,24-octakis[(N,N-dimethylamino)ethylsulfanyl]-phthlocyanine; zinc phthalocyanine-4,4',5",5"'-tetrasulfonic acid; aluminum phthalocyanine chloride; aluminum phthalocyanine hydroxide; aluminum 4,11,18,25-tetrakis(chloro)-phthalocyanine chloride; aluminum 1,8,15,22-tetrakis(phenylthio)-29$H$,31$H$-phthalocyanine chloride; aluminum 2,9,16,23-tetrakis(phenylthio)-29$H$,31$H$-phthalocyanine chloride; aluminum 1,4,8,11,15,18,22,25-octabutoxy-29$H$, 31$H$-phthalocyanine triethylsiloxide; aluminum 1,2,3,4,8,9,10,11,15,16,17,18,22,23,24,25-hexadecachloro-29$H$,31$H$-phthalocyanine; aluminum 1,2,3,4,8,9,10,11,15,16,17,18,22,23,24,25-hexadecachloro-phthalocyanine sulfate; aluminum tetrakis(sulfono)-29H,31H-phthalocyanine chloride; aluminum sulfono-phthalocyanine hydroxide; aluminum 1,8-bis(sulfono)-phthalocyanine hydroxide; aluminum 1,8,15-tri(sulfono)-phthalocyanine hydroxide; silicon phthalocyanine dichloride; silicon phthalocyanine dihydroxide; methylsilicon phthalocyanine chloride; silicon 2,9,16,23-tetra-tert-butyl-29$H$,31$H$-phthalocyanine dihydroxide; silicon 2,3,9,10,16,17,23,24-octakis(octyloxy)-29$H$,31$H$-phthalocyanine dihydroxide; silicon phthalocyanine dihydroxide bis(trihexylsilyloxide); silicon phthalocyanine bis-(4-tert-butyl)benzoate; silicon phthalocyanine bis-(3-thienyl)acetate; silicon phthalocyanine bis-(2-methoxyphenyl)acetate; silicon phthalocyanine bis-(3-methoxyphenyl)acetate; silicon phthalocyanine bis-(4-methoxyphenyl)acetate; silicon phthalocyanine bis-(2,5-dimethoxyphenyl)acetate; silicon phthalocyanine bis-(3,4-dimethoxyphenyl)acetate; silicon phthalocyanine bis-(3,4,5-trimethoxyphenyl)acetate; silicon phthalocyanine bis-(3,4-dimethoxy)benzoate; silicon phthalocyanine bis-3-(3,4-dimethoxyphenyl)propanoate; silicon phthalocyanine bis-4-(3,4-dimethoxyphenyl)butanoate; hydroxysilicon phthalocyanine (bis-methylamino)-hexylsilyloxide; hydroxysilicon phthalocyanine (bis-methylamino)-pentylsilyloxide; hydroxysilicon phthalocyanine (bis-methylamino)-butylsilyloxide; hydroxysilicon phthalocyanine (bis-methylamino)-propylsilyloxide; hydroxysilicon phthalocyanine (bis-methylamino)-ethylsilyloxide; hydroxysilicon phthalocyanine (bis-methylamino)-methylsilyloxide; silicon phthalocyanine bis-methyloxyethyleneoxide; cobalt phthalocyanine; cobalt 1,2,3,4,8,9,10,11,15,16,17, 18,22,23,24,25-hexadecafluoro-29$H$,31$H$-phthalocyanine; copper phthalocyanine; copper 1,8,15,22-tetrakis(sulfono)-phthalocyanine; copper 2,9,16,23-tetra-tert-butyl-29$H$,31$H$-phthalocyanine; copper 3,10, 17,24-tetra-tert-butyl-1,8,15,22-tetrakis(dimethylamino)-29$H$,31$H$-phthalocyanine; copper 1,4,8,11,15,18,22,25-octabutoxy-29$H$,31$H$-phthalocyanine; copper 2,3,9,10,16,17,23,24-octakis(octyloxy)-29$H$,31$H$-phthalocyanine; copper tetrakis(4-cumylphenoxy)-phthalocyanine; copper 1,2,3,4,8,9,10,11, 15,16,17,18,22,23,24,25-hexadecafluoro-29$H$, 31$H$-phthalocyanine; poly(copper phthalocyanine); dilithium phthalocyanine; gallium phthalocyanine chloride; gallium phthalocyanine hydroxide; iron phthalocyanine; iron phthalocyanine chloride; iron 2,9,16,23-tetrakis(sulfono)-phthalocyanine; lead phthalocyanine; lead tetrakis(4-cumylphenoxy)-phthalocyanine; magnesium phthalocyanine; manganese phthalocyanine; manganese phthalocyanine chloride; nickel phthalocyanine; nickel 3,10,17,24-tetrakis(sulfono)-phthalocyanine hydroxide; nickel 4,11,18,25-tetrakis(sulfono)-phthalocyanine hydroxide; nickel 1,4,8,11,15,18,22,25-octabutoxy-29$H$,31$H$-phthalocyanine; tin phthalocyanine oxide; titanyl phthalocyanine; titanium phthalocyanine dichloride; vanadyl 2,9,16,23-tetraphenoxy-29H,31H-phthalocyanine; vanadyl 3,10,17,24-tetra-tert-butyl-1,8,15,22-tetrakis(dimethylamino)-29$H$,31$H$-phthalocyanine; other possible metals: Pd, Ge, Ru, Pt, Lu, Gd.

Naphthalocyanines are selected from the group of 2,3-naphthalocyanine; 2,11,20,29-tetra-tert-butyl-2,3-naphthalocyanine; 5,9,14,18,23,27,32,36-octabutoxy-2,3-naphthalocyanine; boron sub-2,3-naphthalocyanine chloride; cobalt 2,3-naphthalocyanine; copper 2,3-naphthalocyanine; copper 5,9,14,18,23,27,32,36-octabutoxy-2,3-naphthalocyanine; gallium 2,3-naphthalocyanine chloride; nickel 5,9,14,18,23,27,32,35-octabutoxy-2,3-naphthalocyanine; silicon 2,3-naphthalocyanine dichloride; silicon 2,3-naphthalocyanine dihydroxide; silicon 2,3-naphthalocyanine dioctyloxide; silicon 2,3-naphthalocyanine bis(trihexylsilyloxide); tin 2,3-naphthalocyanine; vanadyl 2,3-naphthalocyanine; vanadyl 2,11,20,29-tetra-tert-butyl-2,3-naphthalocyanine; zinc 2,11,20,29-tetra-tert-butyl-2,3-naphthalocyanine.

As described above, a further advantage of using a photosensitizer is that in addition to the above triggering effects, reactive oxygen species are formed that may further damage the endothelial cells lining the vessel wall, thus leading to exacerbated thrombus formation.

## PROCOAGULANTS - ANIONIC LIPOSOMES

Another mediator of the coagulation cascade is phosphatidylserine (PS), an anionic phospholipid that is asymmetrically distributed in the cytosolic membrane leaflet in resting platelets through the action of an ATP-dependent translocase. Upon activation, platelets shed microscopic particles referred to as platelet-derived microparticles that have lost the asymmetrical PS distribution, as a consequence of which PS is translocated to the exocytosolic membrane leaflet. The platelet-derived microparticles provide an anionic milieu required for the propagation of coagulation via the prothrombinase complex **(Figure 2)**. PS binds to two sites on prothrombin, two sites on fXa, and four sites on fVa to induce conformational changes in these proteins that are instrumental in the procession of coagulation. In this respect, studies have shown that anionic liposomes composed of PS [Chiu GN et al. Biochim Biophys Acta. 2003 Jun 27;1613 (1-2):115-21], phosphatidic acid, and to a lesser extent phosphatidylglycerol (PG) and phosphatidylinositol (PI) [Jones ME et al. Thromb Res. 1985 Sep 15;39(6):711-24] are capable of mediating coagulation and the formation of thrombin.

In a further embodiment, liposomes composed of anionic phospholipids selected from the group of PS, PA, PG, and PI are used for the prothrombotic component of SSPLT with the specific purpose of facilitating laser-induced coagulation via the prothrombinase complex. This class of liposome **(Figure 4)** may encapsulate or have engrafted any of the antifibrinolytics, procoagulants, and platelet agonists enabled in the invention, and may be sterically stabilized as described above. In a preferred embodiment, the steric stabilization is achieved by incorporation of 2-6 mol% of photocleavable PEG so as to render the anionic membrane accessible to the clotting factors upon dePEGylation.

## PROCOAGULANTS - PE LIPOSOMES

Initiation of the contact activation pathway occurs when PK, HMWK, fXI, and fXII are exposed to a negatively charged surface. This can occur as a result of interaction with the phospholipids (primarily phosphatidylethanolamine, PE) of circulating lipoprotein particles such as chylomicrons and very-low-density lipoproteins (VLDLs) [Klein S, Arterioscler Thromb Vasc Biol. 2001 Oct;21(10):1695-700].

In a further embodiment, liposomes that mimic the PE-enriched chylomicrons and VLDLs by the incorporation of PE as a membrane constituent can be used to enhance thrombosis by initiating the contact activation pathway. This class of liposomes may encapsulate or have engrafted any of the antifibrinolytics, procoagulants, and platelet agonists enabled in the invention, and may be sterically stabilized as described above. In a preferred embodiment, the PE-containing liposomes of the DDS of choice are sterically stabilized by incorporation of 2-6 mol% of photocleavable PEG so as to render the liposomal surface accessible to PK, HMWK, and the respective clotting factors upon dePEGylation.

## PROCOAGULANTS - $Ca^{2+}$ CONTAINING LIPOSOMES

The procession of coagulation is highly dependent on the presence of extracellular calcium **(Figure 2)**. The blood coagulation factors II, VII, IX, and X bind to the negatively charged membrane (i.e., PS) of activated platelets via calcium ions to constitute the fVIIa-tissue factor complex and the tenase and prothrombinase complexes. Moreover, the platelet kinetics in thrombus development are influenced by supraphysiological concentrations of extracellular calcium in that calcium exaggerates ADP-induced platelet aggregation via a positive feedback mechanism involving $TXA_2$ synthesis [Hu H et al. Thromb Res. 2005;116(3):241-71.

In a further embodiment, calcium is singularly encapsulated or co-encapsulated with any of the antifibrinolytics, procoagulants, and platelet agonists enabled in the invention into the DDS of choice **(Figure 4).** In a preferred embodiment, calcium is encapsulated in liposomes containing anionic phospholipids that are sterically stabilized as described above. The steric stabilization is preferably achieved by incorporation of 2-6 mol% of photocleavable PEG so as to render the anionic membrane accessible to the clotting factors upon dePEGylation.

## *PLATELET AGONISTS*

In addition to or instead of the antifibrinolytic and procoagulant DDS, an agent may be encapsulated that is prothrombotic by exerting an effect on the primary hemostatic system. In the classical sense, primary hemostasis is initiated by platelet adhesion at sites where the endothelium has been perturbed. The adhesion process is mediated by the glycoprotein Ib-IX-V complex and vWF (in areas of high shear) and glycoprotein Ia/IIa and fibrinogen (in areas of low shear). The adhesion of platelets to the vessel wall is ensued by platelet activation, characterized by morphological changes of the cell, expression of glycoprotein IIb/IIIa and P-selectin on the cell surface, and the release of alpha and dense granule constituents (including platelet factor 4, clotting factors such as thrombospondin, fibronectin, and vWF, and primary/secondary hemostatic agonists such as ADP, serotonin, and ionized calcium). In addition, platelets synthetize and release thromboxane $A_2$ ($TXA_2$) and platelet activating factor (PAF), which are potent platelet activators. The liberation of ADP, serotonin, $TXA_2$, and PAF thus promotes activation and recruitment of additional platelets, which occurs in conjunction with thrombin as a product of the coagulation pathway. Platelet aggregation is primarily mediated by the binding of fibrinogen to glycoprotein IIb/IIIa on adjacent platelets. As postulated above, the initial trigger for primary (and secondary) hemostasis is likely laser-induced endothelial damage and the presence of thermally denatured proteins in the thermal coagulum.

In a further embodiment of the invention compounds are encapsulated into the DDS that activate or mediate the propagation of primary hemostasis. Suitable compounds are selected from the group of natural PAF phospholipids with the generic formulas 1-alkyl-2-acetoyl-*sn*-glycero-3-phosphocholine and 1-alkyl-2-hydroxy-*sn*-glycero-3-phosphocholine, synthetic PAFs comprising 1-O-hexadecyl-2-acetoyl-*sn*-glycero-3-phosphocholine, 1-O-octadecyl-2-acetoyl-*sn*-glycero-3-phosphocholine, 1-O-hexadecyl-2-butyroyl-*sn*-glycero-3-phosphocholine, 1-O-octadecyl-2-butyroyl-*sn*-glycero-3-phosphocholine, 1-palmitoyl-2-acetoyl-*sn*-glycero-3-phosphocholine, 1-O-hexadecyl-2-oleoyl-*sn*-glycero-3-phosphocholine, 3-O-hexadecyl-2-acetoyl-*sn*-glycero-1-phosphocholine, 1-O-hexadecyl-2-acetoyl-*sn*-glycerol, 1-O-hexadecyl-2-hydroxy-*sn*-glycero-3-phosphocholine, 1-O-octadecyl-2-hydroxy-*sn*-glycero-3-phosphocholine, 1-O-hexadecyl-2-(homogamma linolenoyl)-*sn*-glycero-3-phosphocholine, 1-O-hexadecyl-2-arachidonoyl-*sn*-glycero-3-phosphocholine, 1-O-hexadecyl-2-eicosapentaenoyl-*sn*-glycero-3-phosphocholine, 1-O-hexadecyl-2-docosahexaenoyl-*sn*-glycero-3-phosphocholine, 1-O-octadecyl-2-O-methyl-*sn*-glycero-3-phosphocholine, 1-O-hexadecyl-2-butenoyl-*sn*-glycero-3-phosphocholine, 1-myristoyl-2-(4-nitrophenylsuccinyl)-*sn*-glycero-3-phosphocholine, ADP, serotonin, $TXA_2$, thrombin.

## *DRUG RELEASE MODALITIES - THERMOSENSITIVE LIPOSOMES*

According to the invention, the drug delivery system is preferably capable of rapidly releasing its contents upon triggering. The drug delivery system may therefore comprise liposomes that are thermosensitive.

Thermosensitive liposomes are a relatively novel class of liposomal DDSs. Hyperthermia has been employed in numerous liposomal formulations in vitro and *in vivo* to initiate a thermotropic alteration in membrane permeability that will lead to rapid, triggered release of the loaded molecules. The heat-induced drug release is centered around the existence of grain boundaries that arise in mixed systems of phospholipids coexisting in the relatively ordered gel (Lβ)- and relatively disordered liquid-crystalline (La)-phases or in monolipidic and mixed systems undergoing main phase transition ($T_m$). Upon reaching $T_m$, the gain in configurational entropy of the lipid chains drives the chain-melting transition that chiefly results in rotational isomerisation (the transition from a trans to gauche conformation) and alterations in the ordering of water molecules (i.e., hydration state of the membrane). This, in turn, results in membrane surface-spanning molecular packing defects whereby polar and charged molecules can transgress the hydrophobic core through thermotropically-induced cavities. Secondly, increases in membrane permeability are correlated to increases in lateral compressibility, i.e., the changes in the cross-sectional area of lipid chains (or volume per lipid molecule) near and at the $T_m$. According to theoretical models, these critical density oscillations in the bilayer display a maximum at the $T_m$ and lower the transmembrane free energy barrier to diffusion of ions and supposedly lower molecular weight compounds - an effect that has been extrapolated (mathematically) to a temperature range several ˚C below and above $T_m$. The spacing between the polar head groups in the near-critical state exposes the hydrocarbons to $H_2O$, which coincides with the exposure of hemispherical cavities that could thus act as permeability gateways.

According to a preferred embodiment of the invention at least part of the drug delivery platform consists of thermosensitive liposomes. In view of the thermal nature of endovascular damage infliction during photocoagulation, the pharmaceutically active compound that is encapsulated in the aqueous compartment is easily released from the liposomal DDS.

In a first embodiment, the triggering mechanism is thus based on thermosensitivity, in which the liposomes are composed of phospholipids that yield a $T_m$ of the system slightly above body temperature, in particular between about 38˚C and 45˚C, and in particular of about 42˚C. Thermosensitive properties of the liposomal DDS are most preferably derived from the incorporation of 1,2-dipalmitoyl-*sn*-glycero-3-phosphatidylcholine (DPPC) ($T_m$ of 41˚C) as the main component phospholipid. In an alternative embodiment, phospholipids with different phase transition temperatures (i.e., with different head groups and/or acyl chain lengths) can be incorporated into the liposomal formulation to adjust the $T_m$ of the system. It is preferred that DPPC is blended with a molar fraction of lecithins with a variable acyl chain length of $\pm 2$ carbon atoms to ensure ideal mixing of phases and to extend the temperature shoulders of the $T_m$.

In a preferred embodiment, the thermosensitive liposomes are sterically stabilized by incorporation of 2-6 mol% of PEG so as to prolong circulation time.

In addition to the 'traditional' thermosensitive liposomes composed of phosphatidylcholines, lysolecithin-containing thermosensitive liposomes have been found to enhance the release kinetics of compounds included in the liposomes. In another preferred embodiment, a molar fraction of lysophosphospholipids selected from the group of lysophosphatidylcholine, lysophosphatidylethanolamine, lysophosphatidylglycerol, lysophosphatidylserine, lysophosphatidic acid, and lysophosphatides is incorporated into the liposomal DDS to speed up release kinetics.

The temperature in the vessel to be treated can be raised to $T_m$ by a second laser pulse (with the first laser pulse being used for the induction of photocoagulation) or by other heat sources such as an infrared (IR) light or heating pad.

### DRUG RELEASE MODALITIES - PLASMALOGEN LIPOSOMES

In a second embodiment, the triggering mechanism is based on the photooxidatively-induced membrane permeation in plasmalogen-containing liposomes. Plasmalogens such as 1-O-(1'-Z-hexadecenyl)-2-palmitoyl-*sn*-glycero-3-phosphocholine and didiplasmalogens such as 1,2-di-O-(1'-Z-hexadecenyl)-*sn*-glycero-3-phosphocholine form single-chain surfactants upon acid- or oxidative-induced cleavage of the vinyl moiety that leads to membrane destabilization and concomitant release of liposomal contents. The methods employed for synthetizing plasmenylcholine and diplasmenylcholine are well known in the art and for example described in [Rui YJ et al. J Organic Chem. 1994;59(19): 5758-5762] and [Shin J et al. J Org Chem. 2003 Aug 22;68(17):6760-6].

In order to generate $^1O_2$ and ROS, a photosensitizer must be incorporated into the DDS for energy transfer to molecular oxygen. Three different sensitizing agents have been used to date in such a configuration, including ZnPC, tin octabutoxyphthalocyanine, and bacteriochlorophyll a, which produced the fastest photo-initiated release among the sensitizers, eliciting 100% calcein release in less than 20 min. However, any photosensitizer enabled in this invention constitutes a suitable compound to mediate triggered drug release via the photooxidative destabilization of the liposomal membrane.

### DRUG RELEASE MODALITIES - PHOTOPOLYMERIZATION OF MEMBRANE LIPIDS

In a further embodiment, the triggering mechanism is based on the photopolymerization of membrane lipids. Bondurant and O'Brien [J. Am. Chem. Sec. 1998, 120, 13541-13542] showed that cross-linking of membrane incorporated 1,2-bis[10-(2',4'-hexadienoloxy)decanonyl]-*sn*-glycero-3-phosphocholine (bis-SorbPC) as a result of irradiation with UV light could destabilize certain PEG-liposomes and increase the bilayer permeability by up to 28,000-fold [Bondurant B et al. Biochim Biophys Acta. 2001 Mar 9;1511(1):113-22; Spratt T et al. Biochim Biophys Acta. 2003 Apr 1;1611(1-2): 35-43]. Similarly, liposomes containing bis-SorbPC can be destabilized with visible light by the co-encapsulation of 1,1'-dioctadecyl-3-3-3'-3'-tetramethylindocarbocyanine (DiI) or distearoyl indocarbocyanine (DiI C(18)3) into the phospholipid bilayer. This technique and the preparation of liposomes has been described in [Mueller A et al. Macromolecules. 2000 Jun 27;33(13):4799-4804] and [Miller CR et al. FEBS Lett. 2000 Feb 4;467 (1):52-6].

### TARGETING - ANTIBODIES

Preferably, the DDS of the invention is provided with targeting specificity. In a preferred embodiment, homing of the liposomes to the target site can be achieved by the coupling of antibodies, Fab' fragments, or peptides to the DDS of choice **(Figure 4),** preferably by attachment thereof to a chemically modified distal end of a polymer chain used for steric stabilization, such as PEG. The antibodies, Fab' fragments, or peptides are preferably directed against platelet activation-specific epitopes, including CD41 (glycoprotein IIb/IIIa) and CD62P (P-selectin), or against fibrin.

Similarly, activated endothelial cells can be targeted inasmuch as activation of these cells is associated with expression of leucocyte adhesion molecules such as E-selectin, ICAM-1, and VCAM-1, which facilitate leucocyte adhesion to the activated endothelium and subsequent diapedesis. In a further embodiment, the DDS of choice may be targeted to activated endothelial cells in the irradiated tissue volume by the conjugation of antibodies, Fab' fragments, or peptides directed against activated endothelial cell -specific epitopes, including E-selectin, ICAM-1, and VCAM-1.

## TARGETING - PEGYLATED ANIONIC LIPOSOMES

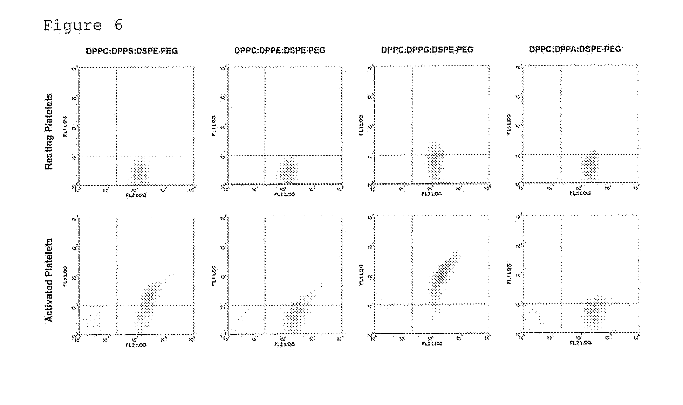

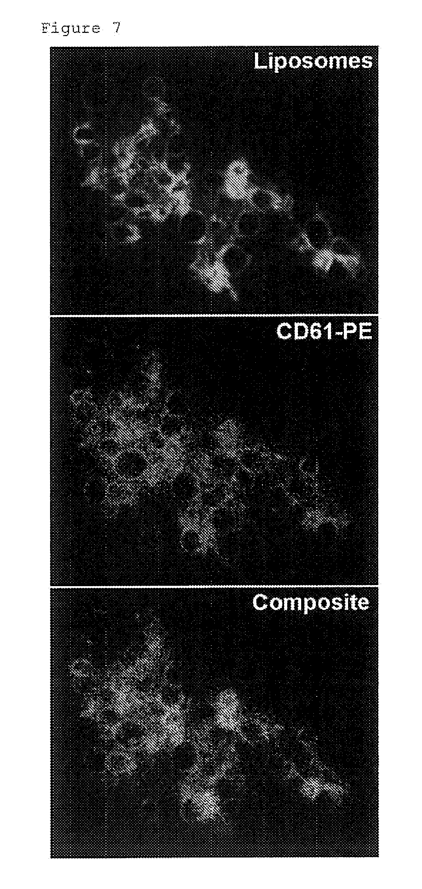

Preferably, phospholipids are incorporated that maintain the thermosensitive properties of the system, that are sterically stabilized, and that have an augmented affinity for activated platelets (and not resting platelets). It was surprisingly found according to the invention that, when liposomes composed of 46 mol% DPPC, 50 mol% 1,2-dipalmitoyl-sn-glycero-3-phosphoglycerol (DPPG), and 4 mol% DSPE-PEG, the DDS of the invention is preferentially targeted to activated platelets, i.e., platelets that are found in thrombi, and not resting platelets **(Figures 6 and 7)**. The same applies, albeit to a lesser extent, to liposomes composed of 46 mol% DPPC, 50 mol% 1,2-dipalmitoyl-sn-glycero-3-phosphoserine (DPPS), and 4 mol% DSPE-PEG **(Figure 6)**. In a preferred embodiment, the targeting of the DDS is achieved by the inclusion of phospholipids of any acyl chain length containing a phosphoglycerol and/or a phosphoserine headgroup and 2-6 mol% DSPE-PEG.

## SSPLT SCOPE OF APPLICABILITY

The invention further relates to the use of a drug delivery system of the invention for the preparation of a medicament for the treatment of vascular and vessel-related pathologies. In a preferred embodiment, the pathology is a port wine stain. Other pathologies in the skin include hemangiomas, telangiectasias, pyogenic granulomas, venous lakes, and angiomas serpiginosum; in ophthalmology vascular or vessel-related anomalies that can be treated with the drug delivery system of the invention with photothermolysis are for example choroidal neovascularization (such as in wet macular degeneration, some forms of chorioretinitis, high myopia, angioid streaks, ocular histoplasmosis), retinal macroaneurysms, intraocular melanomas, retinoblastoma, corneal vascularization, and central serous chorioretinopathy; in gastrointestinal surgery examples of vascular or vessel-related anomalies that can be treated according to the invention are blue rubber bleb nevus syndrome, gastric antral vascular ectasia, radiation proctocolitis, and hereditary hemorrhagic telangiectasia. The drug delivery system of the invention can also be used in oncology for the removal of highly vascularized solid tumors and in brain surgery for the minimally invasive treatment of complex arterio-venous malformations.

## PREFERRED EMBODIMENTS OF THE INVENTION

The following is a summary of the preferred embodiments of the invention as set out in the claims.

The invention thus relates to a drug delivery system for use in the treatment of vascular and vessel-related pathologies, comprising a drug delivery platform that comprises at least one compound capable of exerting an effect on the formation and/or maintenance of a thrombus in the vessel to be treated. Suitably, the drug delivery platform is selected from the group consisting of liposomes, polymeric drug carriers, cells, and cell ghosts.

Preferably the drug delivery platform is sterically stabilized. This can be achieved in various ways. When the platform comprises liposomes, the steric stabilization is effected by grafting of poly(ethylene glycol) onto the liposome surface or by inclusion in the liposomes of covalently linked polymers, diblock copolymers, and/or multiblock copolymers selected from the group of poly(vinyl alcohol) (PVA), polyglycerols, poly(N-vinylpyrrolidone) (PVP) that is activated as succinimidyl ester and bound to the amine-containing anchor (usually PE), poly(N-acryloyl)morpholine (PAcM) that is activated as succinimidyl ester and bound to the amine-containing anchor (usually PE), poly(2-ethyl-2-oxazoline) (PEOZ), poly(2-methyl-2-oxazoline) (PMOZ), polyacrylamide, poly(N-isopropylacrylamide) (NIPAM), poly[N-(2-hydroxypropyl) methacrylamide] (HPMA), poly(styrene-co-maleic acid/anhydride) (SMA), poly(divinyl ether maleic anhydride) (DIVEMA), and/or hydrophobized polysaccharides selected from the group of pullulan, dextran, mannan, and/or polysialic acids, and/or glucuronic acids selected from the group of palmitylglucuronide (PG1cUA), palmitylgalacturoide, and/or gangliosides and sialic acid derivatives selected from the group of monosialoganglioside (GM1), GM3.

When the platform comprises liposomes which are in part composed on anionic constituents, the steric stabilization is effected by the (electro-attractive) adsorption of polymers, diblock copolymers, and/or multiblock copolymers consisting of cationic residues selected from the group of quaternized poly(4-vinylpyridine) (PEVP), poly(ethyleneimine) (PEI), polybetaines (PB).

Suitably, the drug delivery platform comprises liposomes and the compound capable of exerting an effect on the formation and/or maintenance of a thrombus is encapsulated in the aqueous compartment and/or the phospholipid bilayer of the liposome and/or coupled to the steric stabilizer and/or coupled to the lipid bilayer. The compound may be coupled to the distal end and/or to the side chain of the steric stabilizer. Alternatively, the compound is coupled to the lipid bilayer via a linker or anchor.

The compound suitably exerts the effect on the formation and/or maintenance of a thrombus in the vessel to be treated at the level of any of the components of the fibrinolytic pathway, the tissue factor and contact activation pathways (secondary hemostasis), or platelet function (primary hemostasis).

Thus, the compound may be an inhibitor of plasminogen, and is then for example selected from the group of fatty acids which comprises arachidonate, oleate, stearate or from the group of synthetic plasmin(ogen) inhibitors which comprises tranexamic acid (TA), e-aminocaproic acid (ACA), *p*-aminomethylbenzoic acid (AMBA), 4-aminomethyl-bicyclo-2,2,2-octane carboxylic acid (AMBOCA). Alternatively, the compound is an inhibitor of plasmin, which may be selected from the group consisting of purified and/or recombinant $a_2$-antiplasmin, $a_2$-antiplasmin polypeptides, purified and/or recombinant thrombin-activatable fibrinolysis inhibitor TAFI and aprotinin.

In a further embodiment, the compound is an inhibitor of tissue plasminogen activator (tPA) or urokinase-type plasminogen activator (uPA). The inhibitor of tissue plasminogen activator (tPA) is then suitably selected from the group of isolated and purified human PAI1, isolated and purified human PAI2, recombinant human PAI1, recombinant human PAI2, modified human PAI1, modified human PAI2, inhibitory antibodies or derivatives thereof directed against tPA, polypeptides, oligopeptides or short peptides, in particular peptides of 2-10 amino acids, that inhibit tPA and the uPA inhibitor is selected from the group of isolated and purified human PAI1, isolated and purified human PAI2, recombinant human PAI1, recombinant human PAI2, modified human PAI1, modified human PAI2, inhibitory antibodies or a derivative thereof directed against uPA, inhibitory antibodies or derivatives thereof directed against the uPA receptor (uPAR), polypeptides, oligopeptides or short peptides, in particular peptides of 2-10 amino acids, that inhibits uPA. When the compound is an agonist of PAI1 or PAI2, the agonist for PAI1 is a synthetic peptide derived from the fragment $S^{362}$-$A^{380}$ of vitronectin, referred to as BP4, or a synthetic peptide that promotes PAI1 secretion by binding to proteinase-activated receptor-1 (PAR-1), in particular SFLLRN and the agonist for PAI2 is a synthetic peptides that promotes PAI2 secretion, in particular SLIGKV, which binds to proteinase-activated receptor-2 (PAR2), or synthetic peptides that prevent PAI2 polymerization under physiological conditions, in particular TEAAAGTGGVMTG (RCL-PAI2) and SEAAASTAVVIAG (RCL-AT).

The drug delivery system may further comprise an agent that induces a procoagulant response by acting on components from the tissue factor and contact activation pathways. Suitably these agents are agonists of the secondary hemostatic system selected from Factor II(a), Factor III (Tissue Factor, Factor V(a), Factor VII(a), Factor VIII(a), Factor IX(a), Factor X(a), Factor XI(a), Factor XII, Factor XIII(a) or mediators of the contact activation pathway selected from the group consisting of prekallikrein (PK), kallikrein, high molecular-weight kininogen (HMWK).

In a further embodiment the drug delivery platform comprises liposomes that further comprise a photosensitizer. Suitably the photosensitizer is selected from the group consisting of phthalocyanines, naphthalocyanines, chlorines, bacteriochlorins, and porphins as illustrated in **Figure 5,** which are optionally substituted with one or more R-groups selected from the group of H, F, $CF(CF_3)_2$, $O(CH_2)nCF_3$, Cl, Br, $CHCH_2$, $(CH_2)nCH_3$, $(CH_2)nCOOH$, $CONH(CH_2)nNH_2$, $(CH_2)nCONHCH_2CH_2NH_2$, $CONH(CH_2)nCH(NH_2)COOH$, $CH_2CONHCH(CH_2COOH)COOH$, $O(CH_2)nCH_3$, $S(CH_2)nN$ $(CH_3)_2$, $SO_2NH(CH_2)nCH_3$, $SO_2NH(CH_2)nN(CH_3)_2$, $SO_2N[(CH_2)nCH_3]_2$, $SO_2NHCH_2CH(CH_2CH_3)(CH_2)nCH_3$, $C(CH_3)_3$, $OC[(CH_2)nCH_3]_3$, $OCH[CH[CH_3]_2]_2$, $O(CH_2)nN(CH_3)_2$, $O(CH_2)nN(CH_3)_3$, $SC_6H_5$, $OC_6H_5$, $O(C_6H_4)C[(CH_3)_2]$ $(C_6H_5)$, $O(C_6H_3)(COOH)_2$, $O(C_6H_3)[COO(CH_2)nCH_3]_2$, $C_6H_5$, $SO_2NHCH(CH_3)CH_2(C_6H_3)(OCH_3)_2$, $SO_3$, $SO_2Cl$, $N(CH_3)_2$, COOH, $NO_2$, $CH_3$, $CONH_2$, $CH_2NH_2$, and the R'-group is selected from the group of H, $CH_3$, AlCl, AlOH, $AlOSi(CH_3)_3$, $AlOSO_3$, Co, Cu, Li, GaOH, GaCl, Fe, FeCl, $FeO_2$, Pb, Mg, Mn, MnCl, $SiCH3Cl$, $Si(OH)2$, $Si(Cl)2$, $Si\{OC[(CH_2)nCH_3]_3\}_2$, $Si[COCO(C_6H_4)(CH_2)nCH_3]_2$, $Si[OSi(CH_3)_2(CH_2)nN(CH_3)_2]OH$, $Si[O(CH_2)nOCH_3]_2$, $Si[OSi(CH_3)_2(CH_2)nN(CH_3)_2$, $Si[OSi(CH_2)nCH_3]_2$, $SiCH_3$, $Si[OSi(CH_3)_2C(CH_3)_2C(CH_3)_2]_2$, Ni, SnO, $Sn(Cl)_2$, $Ti(Cl)_2$, TiO, VO, Zn, Ag, Cd, Ge, InCl.

Also, the drug delivery platform may be provided with targeting molecules. The targeting molecules can be antibodies or derivatives thereof, in particular Fab' fragments, which are preferably directed against platelet epitopes or fibrin. The platelet epitope may then be CD41 or CD62P.

In a specific embodiment, the drug delivery platform is formed by liposomes and the head group of the lipid is selected from the group consisting of: phosphatidylcholine, phosphocholine, phosphatidylethanolamine, phosphatidic acid, phosphatidylglycerol, phosphatidylserine, phosphatidylethanolamine, phosphatidylinositol, sphingomyelin, diglycerophosphate, glycerol, ethylene glycol, galloylglycerol and glycero-3-succinate. The acyl chain of the lipid is preferably selected from the group consisting of: tridecanoyl (13 carbons), myristoyl (14 carbons), myristoleoyl (14 carbons, cis-alkene at $\Delta_9$), myristelaidoyl (14 carbons, trans-alkene at $\Delta_9$), pentadecanoyl (15 carbons), palmitoyl (16 carbons), palmitoleoyl (16 carbons, cis-alkene at $\Delta g$), palmitelaidoyl (16 carbons, trans-alkene at $\Delta_9$), phytanoyl (16 carbons, methylated at $\Delta_{3,7,11,15}$), heptadecanoyl (17 carbons), stearoyl (18 carbons), petroselinoyl (18 carbons, cis-alkene at $\Delta_6$), oleoyl (18 carbons, cis-alkene at $\Delta_9$), elaidoyl (18 carbons, trans-alkene at $\Delta_9$), linoleoyl (18 carbons, cis-alkenes at $\Delta_{9,12}$), linolenoyl (18 carbons, cis-alkenes at $\Delta_{9,12,15}$), nonadecanoyl (19 carbons), arachidoyl (20 carbons), eicosenoyl (20 carbons, cis-alkene at $\Delta_{11}$), arachidonoyl (20 carbons, cis-alkenes at $\Delta_{5,8,11,14}$), heniecosanoyl (21 carbons), behenoyl (22 carbons), erucoyl (22 carbons, cis-alkene at $\Delta_{13}$), docosahexaenoyl (22 carbons, cis-alkenes at $\Delta_{4,7,10,13,16,19}$), trucisanoyl (23 carbons), lignoceroyl (24 carbons), nervonoyl (24 carbons, cis-alkene at $\Delta_{15}$). The lipids have either a monoacyl (1-acyl-2-hydroxy-*sn*-glycero-3-head group) or diacyl (1-acyl-2-acyl-*sn*-glycero-3-head group) configuration.

In a further embodiment, the drug delivery platform comprises liposomes and at least part of the phospholipids that constitute the liposomes are dipalmitoyl phosphatidyl glycerol (DPPG).

The drug delivery platform may also comprise liposomes that are thermosensitive. These liposomes may thus comprise phospholipids with a phase transition temperature above body temperature, in particular a phase transition temperature between about 37°C and about 45°C, in particular of about 41°C.

In a preferred embodiment at least part of the phospoholipids that constitute the liposomes are dipalmitoyl phosphatidylcholine (DPPC).

The invention further relates to use of a drug delivery system as claimed for the preparation of a medicament for the treatment of vascular and vessel-related pathologies. The pathology is usually a port wine stain but may also be pathologies in the skin such as hemangiomas, telangiectasias, pyogenic granulomas, venous lakes, and angiomas serpiginosum; anomalies in ophthalmology such as choroidal neovascularization (such as in wet macular degeneration, some forms of chorioretinitis, high myopia, angioid streaks, ocular histoplasmosis), retinal macroaneurysms, intraocular melanomas, retinoblastoma, corneal vascularization, and central serous chorioretinopathy; anomalies in gastrointestinal surgery, such as blue rubber bleb nevus syndrome, gastric antral vascular ectasia, radiation proctocolitis, and hereditary hemorrhagic telangiectasia.

In addition to the above, the invention can be practiced without using a drug delivery platform by administering the compound capable of exerting an effect on the formation and/or maintenance of a thrombus in the vessel to be treated. A suitable example is administration of the antifibrinolytic agent tranexamic acid (TA) in unencapsulated form. Suitably, this compound is used in adjuvant amounts.

The invention will be further illustrated in the examples that follow and that are not intended to limit the invention in any way.

In the Examples reference is made to the following figures and tables: Figures 1-11, Tables 1-2.

## FIGURE AND TABLE LEGENDS

**Figure 1:** Aggregation of 5,6-carboxyfluorescein (CF)-labeled platelets at the site of laser-induced damage in hamster dorsal skin fold venules as visualized by intravital fluorescence microscopy without (A-F) and with (G-L) priorly infused heparin (Hep). The bright ellipse in (A) is the laser spot. A=arteriole, V=venule, arrows indicate direction of flow. The time relative to the laser pulse is indicated in the upper right corner (min:sec). The arrowhead in (B) indicates a region of residual hyperfluorescence as a result of heat-mediated CF release from thermosensitive liposomes. The arrowhead in (G) points to a remnant thermal coagulum. The mean±SD lesional sizes with minima (Min) and maxima (Max) are plotted as a function of time for carboxyfluorecein-labeled platelets (M) and carboxyfluorecein-labeled platelets in the presence of heparin (N). In (O), the relative lesional growth is depicted as a function of time. The vertical lines indicated growth peaks for CF (light) and CF+Hep (dark).

**Figure 2:** Secondary hemostasis: the contact activation, tissue factor, and common pathways of coagulation. The contact activation pathway is initiated when prekallikrein, high-molecular-weight kininogen, factor XI and factor XII are exposed to a negatively charged surface. The tissue factor pathway is initiated at the site of injury in response to the release of tissue factor (TF, fIII). Roman numerals represent the respective coagulation factor, whereby "a" indicates an activated state. Factor XII and TF require anionic phospholipids (e.g., phosphatidylserine and phosphatidylinositol) to propagate coagulation. The presence of anionic phospholipids is also required for the formation of the tenase, prothrombinase, and the TF-VIIa complexes. The thrombus, which forms as a result of platelet aggregation (primary hemostasis) and the formation of fibrin, is enzymatically cleaved into fibrin degradation products as a consequence of fibrinolysis. Fibrinolysis is initiated by the conversion of plasminogen to plasmin by tissue-type plasminogen activator (tPA) and urokinase-type plasminogen activator (uPA) - a process that is inhibited by plasminogen activator inhibitors (PAI)1 and 2, and fXIa, fXIIa, and kallikrein (formed from prekallikrein, PK, through fXIIa). The proteolytic breakdown of the thrombus by plasmin is in turn inhibited by α2-antiplasmin (α-2-AP), α2-macroglobulin (α-2-M), and thrombin-activatable fibrinolysis inhibitor (TAFI). Physiological anticoagulants are delineated in the upper right corner and comprise antithrombin III (ATIII), protein C (PC) complexed with protein S (PS) as cofactor, protein Z (PZ), protein Z-dependent protease inhibitor (ZPI), and tissue factor pathway inhibitor (TFPI).

**Figure 3:** Principles of conventional selective photothermolysis (SP) vs. site-specific pharmaco-laser therapy (SSPLT). In SP, irradiation of refractory port wine stain (PWS) vessels with a yellow laser (A) results in semi-obstructive photocoagulation (B, insert) and thrombosis (B). Within 10 min, the thrombus has deteriorated due to fibrinolysis and increased shear stress (C), resulting in a suboptimal damage profile for lesional blanching (D: 1, thermal coagulum; 2, thrombus; 3, patent lumen). SSPLT is an alternative treatment modality for refractory PWS, whereby SP is combined with the systemic administration of a prothrombotic- and/or antifibrinolytic-containing drug carrier. Upon laser irradiation (E), the drug carrier accumulates in the thrombus (F) and its contents are released by a second stimulus (e.g., heat) (G), resulting in local hyperthrombosis and corollary occlusion of the vascular lumen (H). The consequent damage profile (I) conforms to an optimal lesional blanching prognosis.

**Figure 4:** Generic scheme of possible liposomal formulations for site-specific pharmaco-laser therapy. The possible liposomal formulations have been divided into 4 main categories: conventional liposomes, anionic liposomes, sterically stabilized liposomes, and targeted liposomes. Each main category may encompass any of the following subcategories: A) types of drugs: 1. hydrophilic drugs (e.g., tranexamic acid); 2. hydrophobic drugs (e.g., photosensitizers); 3. functionalized photosensitizers; 4. ions (e.g., calcium); B) drug grafting methods: 5. (covalent) attachment to a component (phospho)lipid; 6. (covalent) attachment to an anchor molecule (e.g., cholesterol); 7. (covalent) attachment to a polymer side chain (e.g., polyethylene glycol, PEG); 8. (covalent) attachment to a functionalized distal end of a polymer; C) membrane composition: 9. phosphatidylcholines; 10. phosphatidylcholines with a molar fraction of anionic (phospho)lipids; D) methods of steric stabilization: 11. single chain polymer (e.g., PEG); 12. multichain polymer; 13. multiblock copolymer (e.g., di- or triblock copolymers); 14. photocleavable polymers (e.g., PEGylated plasmalogens); 15. adsorbable polymer (onto anionic membrane surface); E) methods of targeting: 16. antibodies; 17. antibody fragments (e.g., Fab' fragments); 18. peptides. The main categories are not mutually exclusive; e.g., sterically stabilized liposomes may contain anionic membrane constituents as well as antibodies for targeting.

**Figure 5:** Molecular structures of photosensitizers that can be encapsulated in the DDS of choice. The parent structures are presented in the left column, and the substituted derivatives are presented in the right column.

**Figure 6:** Flow cytograms of phycoerythrin-labeled CD61 (CD61-PE)-stained (FL2) resting (top row) and convulxin-activated human platelets (bottom row) incubated for 30 min with 2 mM carboxyfluorescein-encapsulating (FL1) DPPC:DPPS:DSPE-PEG (46:50:4 molar ratio), DPPC:DPPE:DSPE-PEG (46:50:4), DPPC:DPPG:DSPE-PEG (46:50:4), and DPPC:DPPA:DSPE-PEG (46:50:4) large unilamellar vesicles prepared by extrusion technique (LUVET, ~200 nm in diameter). The presence of a discrete platelet population in the upper right corner signifies interaction between platelets and LUVET, as was observed for DPPG-containing LUVETs and, to a lesser extent, for DPPS-containing LUVETs. DPPC, 1,2-dipalmitoyl-*sn*-glycero-3-phosphocholine; DPPS, 1,2-dipalmitoyl-*sn*-glycero-3-phosphoserine; DSPE-PEG, 1,2-distearoyl-*sn*-glycero-3-phosphoethanolamine-polyethylene glycol; DPPE, 1,2-dipalmitoyl-*sn*-glycero-3-phosphoethanolamine; DPPG, 1,2-dipalmitoyl-*sn*-glycero-3-phosphoglycerol; DPPA, 1,2-dipalmitoyl-*sn*-glycero-3-phophatidic acid.

**Figure 7:** Confocal microscopy image of an in vitro induced thrombus incubated for 10 min with 2 mM carboxyfluorescein (CF)-encapsulating DPPC:DPPG:DSPE-PEG (46:50:4) large unilamellar vesicles prepared by extrusion technique (LUVET, ~4.8 mM final lipid concentration) and counterstained by phycoerythrin-labeled CD61 (CD61-PE, red). The colocalization of the green fluorescence from the LUVET-encapsulated CF and the red fluorescence from the CD61-PE-labeled platelets corroborates the interaction between the platelets and LUVETs as observed by flow cytometry in Figure 5.

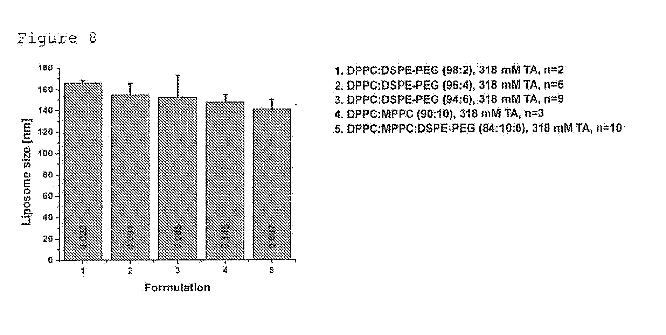

**Figure 8:** Liposome size (y-axis) and polydispersity (inside bars) plotted for several tranexamic acid-containing PEGylated thermosensitive formulations prepared as described in Example 1.2.

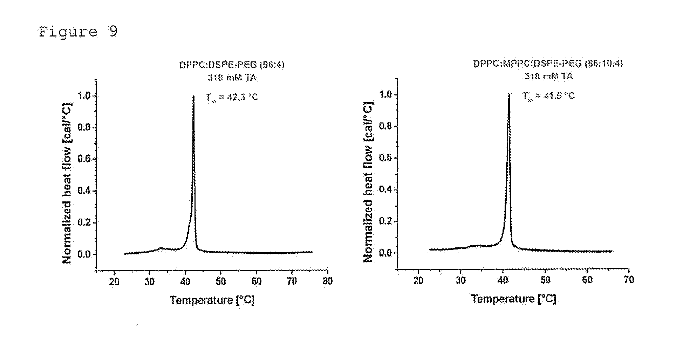

**Figure 9:** Thermograms of tranexamic acid-encapsulating DPPC:DSPE-PEG (96:4 molar ratio) and DPPC:MPPC:DSPE-PEG (86:10:4) large unilamellar vesicles prepared by extrusion technique that comprise candidate formulations for antifibrinolytic SSPLT.

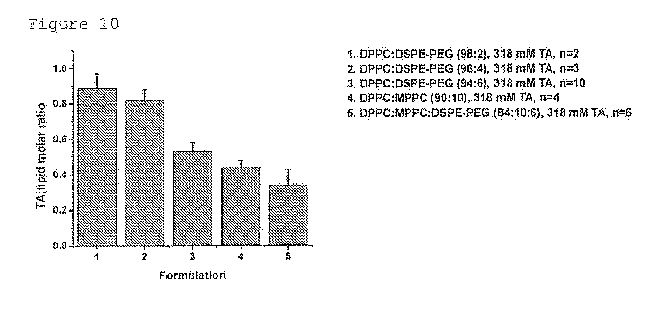

**Figure 10:** Tranexamic acid:lipid ratios of several tranexamic acid-containing PEGylated thermosensitive formulations prepared as described in Example 1.2.

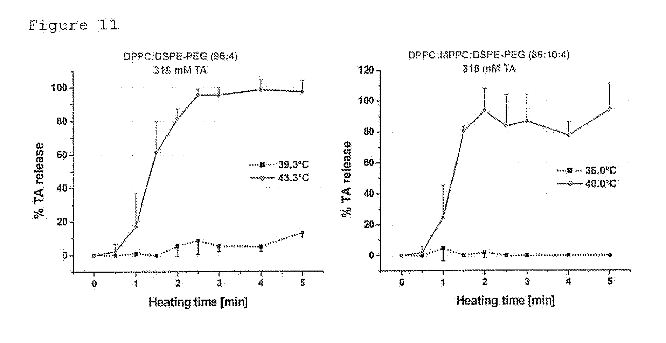

**Figure 11:** Left panel: heat-induced tranexamic acid (TA) release from DPPC:DSPE-PEG (96:4) large unilamellar vesicles prepared by extrusion technique (LUVETs) plotted vs. heating time at 39.3°C (black, dotted line) and 43.3°C (grey, solid line). Right panel: heat-induced TA release from DPPC:MPPC:DSPE-PEG (86:10:4) LUVETs plotted vs. heating time at 36.0°C (black, dotted line) and 40.0°C (grey, solid line). Released TA concentration is expressed as a means±SD percentage of total liposomal TA concentration.

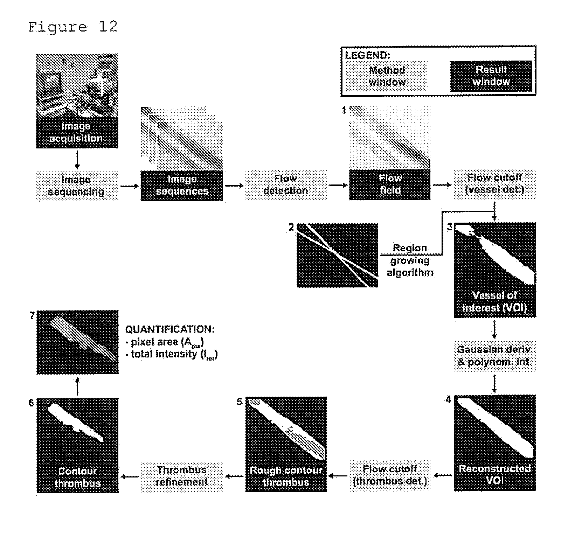

**Figure 12:** Overview of the steps used in the computational image analysis procedure. A set of 125 frames was compared to produce a map corresponding to the flux of platelets **(1)**. This was then combined with a seed image **(2)** to produce the first approximation of the vessel **(3).** The missing part of the vessel was reconstructed using intensity gradients and polynomial interpolation **(4).** The portions in the vessel with a minimum flow were selected by using the cutoff value **(5)**. After assigning two probabilities to every pixel in the detected thrombus and the detected vessel wall **(6),** the final contours of the thrombus could be defined **(7)**. Abbreviations (in chronological order): det. =detection; deriv.=derivative; polynom.=polynomial; int.=interpolation.

**Table 1:** The mean encapsulation efficiency ($E_{eff}$), trapped volume ($V_t$)), and endovesicular tranexamic acid concentration ($C_{TA}$) of several tranexamic acid-containing PEGylated thermosensitive formulations prepared as described in Example 1.2.

**Table 2:** Fundamental properties of several tranexamic acid (TA)-containing PEGylated thermosensitive formulations, prepared as described in Example 1.2, that were used to calculate parameters such as the trapped volume per vesicle ($eV_t$) and the endovesicular TA concentration ($C_{TA}$, Table 1). DPPC, 1,2-dipalmitoyl-*sn*-glycero-3-phosphocholine; DSPE-PEG, 1,2-distearoyl-*sn*-glycero-3-phosphoethanolamine-polyethylene glycol; MPPC, 1-palmitoyl-2-hydroxy-*sn*-glycero-3-phosphocholine; OM, outer membrane; IM, inner membrane.

## EXAMPLES

### EXAMPLE 1

Preparation of the drug delivery system of the invention

Several possible combinations of drug delivery systems that can be employed in SSPLT are presented in **Figure 4.** In the following the preparation of the various types of liposomal drug delivery systems is described.

1. *Thermosensitive liposomes encapsulating an antifibrinolytic agent (tranexamic acid)*

1,2-dipalmitoyl-*sn*-glycero-3-phosphatidylcholine (DPPC) and 1-palmitoyl-2-hydroxy-*sn*-glycero-3-PC (MPPC) were obtained from Avanti Polar Lipids (Alabaster, AL). HEPES [4-(2-hydroxyethyl)-1-piperazineethanesulfonic acid] sodium salt was acquired from Sigma Aldrich (St. Louis, MO). Tranexamic acid (4-(aminomethyl)cyclohexane-1-carboxylic acid, TA) was purchased from Fluka (Buchs, Switzerland). All other reagents were analytical grade.
Large unilamellar vesicles prepared by extrusion technique (LUVETs) were prepared from DPPC:MPPC in a 90:10 molar ratio. DPPC was dissolved in chloroform and MPPC in chloroform:methanol (4:1 ratio) and mixed at the abovmentioned ratio. The solution was desiccated by evaporation under a stream of $N_2$ gas and exsiccated for 20 min in a vacuum exsiccator. The resulting lipid film was hydrated with 318 mM TA in 10 mM HEPES buffer (pH 7.4, osmolarity 0.302 osmol/kg) to a final lipid concentration of 5 mM and bath sonicated for 10 min. The mixture was subjected to 10 freeze-thaw cycles and extruded 5 times through 0.2 $\mu$m filters (Anotop, Whatman, Brentford, UK) at 55°C by keeping the tubes containing the samples in a thermostatic water bath. Unencapsulated TA was removed from the LUVET suspensions by size exclusion chromatography during 4 min centrifugation at 100 $\times g$ (2 mL spin columns, gel volume 2.2-2.5 mL, loading volume 200 $\mu$L, Sephadex G-50 fine, GE Healthcare, Chalfont St. Giles, UK). The equilibration buffer, and thus the storage buffer for the LUVET, consisted of 10 mM HEPES and 0.88% (w/v) NaCl, pH 7.4 and an osmolarity of 0.291 osmol/kg. The eluted LUVETs were stored in the dark at 4°C.
The inclusion of MPPC is mandatory so as to prevent liposome aggregation and fusion in the absence of steric stabilization.

2. *PEGylated thermosensitive liposomes encapsulating an antifibrinolytic agent (tranexamic acid)*

1,2-dipalmitoyl-*sn*-glycero-3-phosphatidylcholine (DPPC) and 1-palmitoyl-2-hydroxy-*sn*-glycero-3-PC (MPPC) were obtained from Avanti Polar Lipids (Alabaster, AL). 1,2-distearoyl-*sn*-glycero-3-phosphoethanolamine-polyethylene glycol (DSPE-PEG2000, average PEG molecular mass of 2,000 amu) and HEPES [4-(2-hydroxyethyl)-1-piperazineethanesulfonic acid] sodium salt were acquired from Sigma Aldrich (St. Louis, MO). Tranexamic acid (4-. (aminomethyl)cyclohexane-1-carboxylic acid, TA) was purchased from Fluka (Buchs, Switzerland). All other reagents were analytical grade.
Large unilamellar vesicles prepared by extrusion technique (LUVETs) were prepared in the following compositions: DPPC:DSPE-PEG2000 (98:2, 96:4, and 94:6 molar ratios) and DPPC:MPPC:DSPE-PEG2000 (84:10:6 and 86:10:4). DPPC and DSPE-PEG2000 were dissolved in chloroform and MPPC in chloroform:methanol (4:1 ratio) and mixed at the abovmentioned ratios. The solutions were desiccated by evaporation under a stream of $N_2$ gas and exsiccated for 20 min in a vacuum exsiccator at room temperature (RT). The resulting lipid films were hydrated with 318 mM TA in 10 mM HEPES buffer (pH = 7.4, osmolarity = 0.302 osmol·$kg^{-1}$) to a lipid concentration of 5 mM and bath sonicated for 10 min. The mixtures were subjected to 10 freeze-thaw cycles and extruded 5 times through 0.2 $\mu$m Anopore aluminum oxide filters (Anotop, Whatman, Brentford, UK) at 55°C. The formulations were stored in the dark at 4°C until further use.
Unencapsulated TA was removed from the LUVET suspensions by size exclusion chromatography during 4 min centrifugation at 100 $\times g$ and 4°C in a 2 mL syringe, containing a gel volume of 2.2-2.5 mL (Sephadex G-50 fine, GE Healthcare, Chalfont St. Giles, UK), and using a loading volume of 200 $\mu$L. The equilibration buffer, and thus the storage buffer for the LUVETs, consisted of 10 mM HEPES, 0.88% (w/v) NaCl, pH = 7.4, with an osmolarity of 0.291 osmol·$kg^{-1}$. The eluted LUVETs were stored in the dark at 4°C.

3. *Photosensitizer-containing liposomes*

A preparation method for phosphatidylcholine liposomes encapsulating zinc phthalocyanine (ZnPC) in the phospholipid bilayer has been described in [Ricchelli F et al. Biochim Biophys Acta. 1994 Dec 30;1196(2):165-71] and [de Oliveira CA et al. Chem Phys Lipids. 2005 Jan;133(1):69-78].
A preparation method for phosphatidylcholine liposomes encapsulating functionalized ZnPC in the phospholipid bilayer (e.g., zinc 2,3,9,10,16,17,23,24-octakis[(*N,N*-dimethylamino)ethylsulfanyl]phthlocyanine) has been described in [Vittar NB et al. Int J Biochem Cell Biol. 2008;40(10):2192-205].

A preparation method for PEGylated phosphatidylcholine liposomes encapsulating functionalized aluminum phthalocyanine in the aqueous compartment of the liposome (e.g., aluminum tetrakis(sulfono)-29H,31H-phthalocyanine) has been described in [Derycke AS et al. J Natl Cancer Inst. 2004 Nov 3;96(21):1620-30].

4. *PEGylated zinc phthalocyanine-containing thermosensitive liposomes encapsulating* an *antifibrinolytic agent (tranexamic acid)*

1,2-dipalmitoyl-sn-glycero-3-phosphatidylcholine (DPPC) was obtained from Avanti Polar Lipids (Alabaster, AL). 1,2-distearoyl-sn-glycero-3-phosphatidylethanolamine-polyethylene glycol (DSPE-PEG2000, average PEG molecular mass of 2,000 amu), zinc phthalocyanine (ZnPC), and HEPES (4-(2-hydroxyethyl)-1-piperazineethanesulfonic acid) sodium salt were acquired from Sigma Aldrich (St. Louis, MO). Tranexamic acid (4-(aminomethyl)cyclohexane-1-carboxylic acid, TA) was purchased from Fluka (Buchs, Switzerland). All other reagents were analytical grade.

Large unilamellar vesicles prepared by extrusion technique (LUVETs) were prepared from DPPC:DSPE-PEG2000.in a 96:4 molar ratio containing 5 $\mu$M ZnPC. The phospholipids were dissolved in chloroform and ZnPC was dissolved in pyridine (100 $\mu$M stock solution). Both solutions were mixed at the abovmentioned ratio. The solution was desiccated by evaporation under a stream of $N_2$ gas until a film had formed and exsiccated for 1 h in a vacuum exsiccator. The resulting lipid film was hydrated with 318 mM TA in 10 mM HEPES buffer (pH 7.4, osmolarity 0.302 osmol/kg) to a final lipid concentration of 5 mM and intermittently bath sonicated for 30 s while incubating for 1 h at 55°C. The mixture was subjected to 10 freeze-thaw cycles and extruded 5 times through 0.2 $\mu$m filters (Anotop, Whatman, Brentford, UK) at 55°C by keeping the tubes containing the samples in a thermostatic water bath. Unencapsulated TA was removed from the LUVET suspensions by size exclusion chromatography during 4 min centrifugation at 100 $\times g$ (2 mL spin columns, gel volume 2.2-2.5 mL, loading volume 200 $\mu$L, Sephadex G-50 fine, GE Healthcare, Chalfont St. Giles, UK). The equilibration buffer, and thus the storage buffer for the LUVETs, consisted of 10 mM HEPES and 0.88% (w/v) NaCl, pH 7.4 and osmolarity of 0.291 osmol/kg. The eluted LUVETs were stored in the dark at 4°C.

5. *Desorbable PEGylated anionic liposomes for mediation of prothrombinase complex formation*

The preparation of anionic liposomes, (desorbable) PEGylated anionic liposomes, and (desorbable) anionic liposomes containing functionalized PEG has been described in [Jones ME et al. Thromb Res. 1985 Sep 15;39(6): 711-24], [Chiu GN et al. Biochim Biophys Acta. 2002 Feb 18;1560(1-2):37-50], and [Chiu GN et al. Biochim Biophys Acta. 2003 Jun 27;1613(1-2):115-21], respectively.

6. *PEGylated plasmalogen liposomes encapsulating a photosensitizer and an antifibrinolytic agent (tranexamic acid)*

The preparation of PEGylated photosenstizer-encapsulating plasmalogen liposomes has been described in [Shin J et al. J Control Release. 2003 Aug 28;91(1-2):187-200] and in [Thompson DH et al. Methods Enzymol. 2004; 387:153-68]. Instead of hydrating the lipid film with calcein and buffer solution, respectively, the lipid film was hydrated with a 10 mM HEPES buffer containing 318 mM tranexamic acid (TA) for the preparation of TA-encapsulating liposomes.

Unencapsulated TA was removed from the LUVET suspensions by size exclusion chromatography during 4 min centrifugation at 100 $\times g$ and 4°C in a 2 mL syringe, containing a gel volume of 2.2-2.5 mL (Sephadex G-50 fine, GE Healthcare, Chalfont St. Giles, UK), and using a loading volume of 200 $\mu$L. The equilibration buffer, and thus the storage buffer for the LUVETs, consisted of 10 mM HEPES, 0.88% (w/v) NaCl, pH = 7.4, with an osmolarity of 0.291 osmol·kg$^{-1}$. The eluted LUVETs were stored in the dark at 4°C.

## EXAMPLE 2

Characterization of the drug delivery system of the invention

1. *Determination of physicochemical properties of the liposomal drug delivery system*

Liposome size and polydispersity (a measure of size distribution) were measured by photon correlation spectroscopy at a 90° angle using unimodal analysis (Zetasizer 3000, Malvern Instruments, Malvern, UK) after dilution with equilibration buffer (10 mM HEPES, 0.88% (w/v) NaCl, pH = 7.4, osmolarity of 0.291 osmol·kg$^{-1}$). The results of several candidate formulations are presented in Figure 8.

Liposomal phase transition temperatures were measured by differential scanning calorimetry (MicroCal, Northampton, MA) after dilution of liposomes with equilibration buffer to a 3 mM final lipid concentration. Equilibration buffer was used as reference. The results of two candidate formulations are presented in Figure 9.

2. *Determination of liposomal tranexamic acid:lipid ratio, encapsulation efficiency, trapped volume, trapped volume per vesicle, the quantity of tranexamic acid molecules per vesicle, and endovesicular tranexamic acid concentration*

An assay based on primary amine derivatization with fluorescamine (4-phenylspiro-[furan-2(3*H*),1-phthalan]-3,3'-dione) (Sigma Aldrich, St. Louis, MO) was developed for the quantification of liposomal tranexamic acid (TA) in detergent-treated buffered solutions.

Large unilamellar vesicles prepared by extrusion technique (LUVETs, 5 mM final lipid concentration) were gel filtered as described in Examples 1.1 and 1.2 and diluted 500× with equilibration buffer (10 mM HEPES, 0.88% (w/v) NaCl, pH = 7.4, osmolarity of 0.291 osmol·kg$^{-1}$). 500 μL of the LUVET solution was mixed with 250 μL of 5% TX100 (Fluka, Buchs, Switzerland) (1% final concentration) and 500 μL of 1.08 mM fluorescamine in acetone (432 μM final concentration) [Udenfriend S et al. Science 1972;178(63):871-872]. Following 30 min incubation at 37˚C, the samples were assayed spectrofluorometrically at $\lambda_{ex}$= 391±5 nm and $\lambda_{em}$= 483±5 nm (SPF 500C, American Instrument Company, Silver Springs, MD). Reference standards in the 0-4.0 μM TA concentration range were included in each assay. Liposomal TA concentrations were derived by solving the regression equation of the reference curve for the respective fluorescence emission intensities.

Phospholipid concentrations were determined by the phosphorous assay according to [Rouser G et al. Lipids. 1970;5(5):494-6].

Drug:lipid ratios were calculated by dividing the TA concentration as determined by the fluorescamine assay (corrected for the gel filtration efficiency) by the phospholipid concentration as determined by the Rouser assay. The drug:lipid ratios of several candidate formulations are presented in **Figure 10.**

The encapsulation efficiency, $E_{eff}$, was computed by dividing the liposomal TA:lipid molar ratio by the initial TA:lipid molar ratio (318 mM TA per 5 mM phospholipid, i.e., 63.6) and expressed as a percentage **(Table 1).**

The trapped volume ($V_t$, L·mole$^{-1}$ lipid) was computed with the equation obtained from [N.J. Zuidam, R. de Vrueh, D.J. Crommelin, in: V.P. Torchilin, V. Weissig (Eds.), Liposomes, 2nd Edition, Oxford University Press, Oxford, 2003, pp. 64]:

$$V_t \; = \; (500/3)(A)(N)(r_v)$$

where A is the area of the membrane occupied by one lipid, N is the Avogadro constant (6.022×10$^{23}$ mol$^{-1}$), and $r_v$ the radius of the vesicle (based on photon correlation spectroscopy). The areas per phospholipid molecule were obtained from literature: 49.4 Å$^2$ for DPPC [Nagle JF et al. Curr. Opin. Struc. Biol. 2000;10(4):474-480], 50.0 Å$^2$ for DSPE-PEG [Majewski J et al. J. Am. Chem. Soc. 1998;120(7):1469-1473], and 48.0 Å$^2$ for MPPC [Chi LM et al. Biophys. J. 1990; 57(6):1225-1232]. For phospholipid mixtures, the areas were weighed averages indexed for the molar ratio of each lipid component:

$$A_{weighed} \; = \; [(A_{DPPC})(mol\%_{DPPC})+(A_{DSPE-PEG})(mol\%_{DSPE-PEG})+(A_{MPPC})(mol\%_{MPPC})]/100\%$$

The $A_{weighed}$ was 49.4 Å$^2$ for all MPPC-lacking formulations and 49.3 Å$^2$ for the MPPC-containing formulations. The $V_t$s of several candidate formulations are presented in **Table 1.**

The $V_t$ per vesicle ($eV_t$, expressed in L/vesicle) was derived by extrapolating the quantity of phospholipid molecules per vesicle **(Table 2).** The quantity of phospholipid molecules per vesicle was defined as the cumulative number of lipids in the outer ($l_{om}$) and inner membrane leaflet ($l_{im}$), based on the $A_{weighed}$, the measured vesicle size with radius $r_v$, a bilayer thickness of 3.93 nm [Tahara Y et al. Micron. 1994;25(2):141-149], and a spherical morphology (where area sphere = 4pr$^2$):

$$l_{om} \; = \; 4pr_v{}^2/A_{weighed}$$

$$l_{im} = 4p(r_v-3.93)^2/A_{weighed}$$

The eV$_t$ was calculated by:

$$eV_t = [(l_{om}+l_{im})V_t]/N$$

The quantity of TA molecules per vesicle (Q$_{TA}$) was obtained by multiplying (l$_{om}$+l$_{im}$) by the TA:lipid ratio. Subsequently, the endovesicular TA concentration (C$_{TA}$) was computed from the amount of TA molecules per vesicle for a given eV$_t$:

$$C_{TA} = (Q_{TA}/N)(1/eV_t)$$

The C$_{TA}$s of several candidate formulations are presented in **Table 1.**

3. *Determination* of *liposomal photosensitizer concentration, dimerization equilibrium constants, triplet state properties, and reactive oxygen species production.*

The determination of liposomal zinc phthalocyanine concentration has been described in [de Oliveira CA et al. Chem Phys Lipids. 2005 Jan;133(1):69-78].

The determination of photosensitizer dimerization constants and triplet state properties in liposomal formulations has been described in [Nunes SM et al. Braz J Med Biol Res. 2004 Feb;37(2):273-84].

The determination of reactive oxygen species production by the liposomal photosensitizer has been described in [Hadjur C et al. Journal of Photochemistry and Photobiology B: Biology 1997;38:196-202].

**EXAMPLE 3**

Drug release from the drug delivery system of the invention

1. *Thermally-induced tranexamic acid release from PEGylated thermosensitive liposomes*

Quantification of the heat-induced release of tranexamic acid from thermosensitive large unilamellar vesicles prepared by extrusion technique (LUVETs) was performed for formulations composed of DPPC:DSPE-PEG (96:4 molar ratio) and DPPC:MPPC:DSPE-PEG (86:10:4 molar ratio).

Prior to heat treatment the gel filtered LUVET suspensions were diluted 10× with equilibration buffer that had been kept at 4°C. 20 μL of the gel filtered LUVET suspension was diluted 50-fold (n = 3 per experiment) and assayed spectrofluorometrically for total vesicular TA concentration (final dilution factor of 1250). The mean total vesicular TA concentration was used to calculate the percentage of released TA molecules.

Following 5 min equilibration at 4°C, 160 μL of the LUVETs was suspended in 0.2 mL ultra-thin PCR tubes (Thermowell Gold, Corning, New York, NY) and incubated at 4°C for 10 min before thermally-induced drug release, which was carried out in a thermal cycler (Biozym, Oldendorf, Germany). Active drug release from TA-encapsulating DPPC:DSPE-PEG (96:4) and DPPC:MPPC:DSPE-PEG (86:10:4) LUVETs was induced near the maximum phase transition temperature (T$_m$), namely at 43.3 and 40.0°C, respectively **(Figure 9)**, and 4°C below the T$_m$. Samples were heated for a predefined period, after which they were immediately submersed in an ice bath. The entire volume was then transferred to 0.5 mL polycarbonate ultracentrifuge tubes and centrifuged (Optima TLX Ultracentrifuge, Beckman-Coulter, Fullerton, CA) at 355,000 ×g for 60 min at 4°C to pellet the LUVETs. 50 μL of the supernatant was carefully aspirated and the released TA in the supernatant was quantitated spectrofluorometrically following 50-fold dilution with equilibration buffer (final dilution factor of 1250). Phospholipid analysis of the supernatant showed that at least 99.9% of the phospholipids was pelleted. Four untreated 160-μL LUVET samples were included in the ultracentrifugation step to serve as negative control. These samples were processed in the same manner as the heat-treated samples to determine ultracentrifugation-induced TA leakage.

TA release was calculated by dividing the mean TA concentration in the supernatant of heat-treated samples by the mean total TA concentration in the LUVETs. TA concentrations were corrected for the mean TA content in the supernatant of the ultracentrifugation control samples. The mean$\pm$SD TA concentration in the supernatant of the centrifuge control samples of DPPC:DSPE-PEG (96:4) and DPPC:MPPC:DSPE-PEG (86:10:4) LUVETs was 2.4$\pm$5.1% (n = 48) and 7.1$\pm$11.1% (n = 56) of the total vesicular TA concentration, respectively.

The heat-induced TA release kinetics from DPPC:DSPE-PEG (96:4) and DPPC:MPPC:DSPE-PEG (86:10:4 molar ratio) LUVETs are presented in **Figure 11.**

*2. Photosensitizer-induced release*

The induction and quantification of photo-oxidation-mediated content release from plasmalogen liposomes has been described in [Thompson DH et al. Biochim Biophys Acta. 1996 Feb 21;1279(1):25-34].

## EXAMPLE 4

<u>Targeting mechanisms</u>

1. *Targeting of PEGylated photosensitizer-encapsulating immunoliposomes to the site of laser-induced damage*

For the in vivo studies on the targeting of immunoliposomes to the site of laser-induced damage, a hamster dorsal skin fold model was used in combination with intravital fluorescence microscopy and external laser irradiation as described in [Bezemer R et al., Opt Express 2007 Jul 25;15(4):8493-8506].

PEGylated zinc phthalocyanine (ZnPC)-containing liposomes were prepared as described in Example 1.4. The conjugation of rat anti-mouse CD62P monoclonal antibodies (clone RB40.34, Fitzgerald Industries, Concord, MA) to sterically stabilized liposomes has been described in detail in [A.L. Klibanov, V.P. Torchilin, S. Zalipsky, in: V.P. Torchilin, V. Weissig (Eds.), Liposomes, 2nd Edition, Oxford University Press, Oxford, 2003, pp. 231-263]. Liposomes onto which no antibody was grafted and antibody-lacking liposomes containing no ZnPC served as negative controls.

For fluorescent thrombus staining, platelets were labeled in vivo by the systemic administration of 5,6-carboxyfluorescein in accordance with [Heger M et al. Anal Quant Cytol Histol. In press]. After platelet labeling, 180 $\mu$L of the liposome suspension (10 mM final lipid concentration) was gently infused via the subclavian vein.

Subocclusive thrombi were induced with a frequency-doubled Nd:YAG laser (532 nm, Entertainer, Laser Quantum; Fig. 5, 7) at a power of 224 mW and a mean$\pm$SD incident radiant exposure of 289$\pm$38 J/cm$^2$ at a 2.3$\times$10$^{-3}$ mm$^2$ spot size. The laser was mounted on a translator stage for axial positioning of the beam that was guided at an angle onto the vessel by a mirror in the tip of the laser probe. The pulse duration of 30 ms was regulated with a vibration-controlled analog shutter interposed between the laser aperture and mirror. The laser beam passed through a 10% transmission filter incorporated into the shutter aperture to generate a low power spot size for targeting.

Laser-induced thrombi were visualized using a FITC filter set ($\lambda_{ex}$=480$\pm$15 nm, DC =505 nm, $\lambda_{em}$=535$\pm$20 nm, B2EC, Nikon, Tokyo, Japan), and colocalization of the liposomes with the thrombus was visualized by using a custom-designed filter set ($\lambda_{ex}$=650$\pm$10 nm, model FB650-10, Thorlabs, Newton, NJ; $\lambda_{em}$=700$\pm$40 nm, model FB700-40, Thorlabs; Dichroic mirror=664 nm, model NT47-425, Edmund Optics, Barrington, NJ).

Endovascular events were recorded for a period of 30 min.

For image analysis, software was developed in *Mathematica* (version 6.0.1, Wolfram Research, Champaign, IL) to quantify the CF-labeled lesions on the basis of a set of objective parameters. The program was compiled from a sequence of algorithms depicted in **Figure 12.**

The principal premises that the program is built around include the relatively static nature of the lesion in an environment of dynamic flow. Consequently, the first parameter that was used to discriminate between 'static' and 'dynamic' regions was blood flow, which was detected by calculating the mean absolute time derivative of the pixel intensity, i.e., the number of fluorescently labeled platelets passing through a pixel in sequential frames, over period of 125 frames (5 s) (**Figure 12**, step 1). Subsequently, a region growing algorithm was implemented on a user-defined seed image, where the vessel of interest was manually marked by a line or a cross (**Figure 12,** step 2), and combined with the flow information to demarcate the vessel of interest (**Figure 12,** step 3).

Due to the absence of flow in the laser-induced lesion, the respective no-flow segment of the vessel was excluded from the demarcated vascular structure. A second order Gaussian derivative of the pixel intensity data was therefore used in the direction perpendicular to the vessel's longitudinal axis to reconstruct the missing segment. Additionally, polynomial interpolations were used to eliminate undefined vascular segments and to incorporate missing 'vessel pixels' as a result of inherently poor pixel intensity gradients (**Figure 12**, step 4). After the boundaries of the contralateral vascular walls were defined, the laser-induced lesion was characterized by applying a cut-off value to the flow data, selecting only the segment in the vessel with a minimum of flow (**Figure 12,** step 5). The flow cut-off value was defined by the first zero-crossing of the second derivative of the mean pixel intensity of the lesions. This value was then held constant throughout the analysis of the movie prior to computing the complete set, as was the seed image. In the next step two probabilities were assigned to every pixel, depending on the number of pixels in its vicinity that had been defined as either part of the thrombus or the vessel wall (**Figure 12,** step 6). Subsequently, the thrombus margins near the vessel wall were refined by comparing these probabilities (**Figure 12**, step 7). Finally the contoured lesions are saved in separate image files for quantification of $A_{pix}$ and $I_{tot}$ using SigmaScan Pro (Systat Software, Mountain View, CA).

*1. Targeting of PEGylated anionic liposomes to the site of laser-induced damage*

PEGylated zinc phthalocyanine (ZnPC)-containing anionic liposomes composed of 1,2-dipalmitoyl-sn-glycero-3-phosphatidylcholine (DPPC), 1,2-dipalmitoyl-sn-glycero-3-phosphatidylglycerol (DPPG), and 1,2-distearoyl-sn-glycero-3-phosphatidylethanolamine-polyethylene glycol (DSPE-PEG2000, average PEG molecular mass of 2,000 amu) were prepared in a 46:50:4 molar ratio as described in Example 1.4.

For the in vivo targeting of the liposomes to the site of laser-induced endovascular damage, the protocol as described in Example 4.1 was employed.

Having described preferred embodiments of the invention with reference to the accompanying figures, it is to be understood that the invention is not limited to the precise embodiments, and that various changes and modifications may be effected therein by those skilled in the art without departing from the scope or spirit of the invention as defined in the appended claims.

Table 1

| | Formulation (mol%) | $E_{eff}$ | $V_t$(L·mol$^{-1}$) | $C_{TA}$(M) |
|---|---|---|---|---|
| 1 | DPPC:DSPE-PEG2000 (98:2) | 1.40% | 4.11 | 0.217 |
| 2 | DPPC:DSPE-PEG2000 (96:4) | 1.29% | 3.84 | 0.214 |
| 3 | DPPC:DSPE-PEG2000 (94:6) | 0.83% | 3.78 | 0.140 |
| 4 | DPPC:MPPC (90:10) | 0.70% | 3.72 | 0.119 |
| 5 | DPPC:MPPC:DSPE-PEG2000 (84:10:6) | 0.53% | 3.56 | 0.095 |

Table 2

| | Formulation (mol%) | OM surface (nm) | IM surface (nm) | No of lipids OM | No of lipids IM | Lipids per vesicle | TA molecules per vesicle | oV (L/ vesicle) |
|---|---|---|---|---|---|---|---|---|
| 1 | DPPC: DSPE-PEG2000 (98:2) | 86,500 | 78,498 | 175,059 | 158,864 | 333,923 | 298,165 | $2.28 \times 10^{-18}$ |
| 2 | DPPC: DSPE-PEG2000 (96:4) | 76,167 | 67,720 | 152,085 | 137,019 | 289,104 | 237,516 | $1.84 \times 10^{-18}$ |
| 3 | DPPC: DSPE-PEG2000 (94:6) | 72,804 | 66,478 | 147,269 | 132,451 | 270,720 | 147,848 | $1.75 \times 10^{-18}$ |
| 4 | DPPC: MPPC (90:10) | 68,315 | 61,225 | 135,923 | 121,817 | 257,740 | 114,195 | $1.59 \times 10^{-18}$ |

(continued)

| Formulation (mol%) | | OM surface (nm) | IM surface (nm) | No of lipids OM | No of lipids IM | Lipids per vesicle | TA molecules per vesicle | oV (L/ vesicle) |
|---|---|---|---|---|---|---|---|---|
| 5 | DPPC: MPPC: DSPE-PEG2000 (84:10:6) | 62,474 | 55,703 | 124,213 | 110,750 | 234,963 | 79,027 | $1.39 \times 10^{-18}$ |

**Claims**

1. Drug delivery system for use in the treatment of vascular and vessel-related pathologies, comprising a drug delivery platform that comprises at least one compound capable of exerting an effect on the formation and/or maintenance of a thrombus in the vessel to be treated.

2. Drug delivery system as claimed in claim 1, wherein the drug delivery platform is selected from the group consisting of liposomes, polymeric drug carriers, cells, and cell ghosts.

3. Drug delivery system as claimed in claim 1 or 2, wherein the drug delivery platform is sterically stabilized.

4. Drug delivery system as claimed in claim 3, wherein the platform comprises liposomes and the steric stabilization is effected by grafting of poly(ethylene glycol) onto the liposome surface.

5. Drug delivery system as claimed in any one of the claims 1-4, wherein the platform comprises liposomes and the compound capable of exerting an effect on the formation and/or maintenance of a thrombus is encapsulated in the aqueous compartment and/or the phospholipid bilayer of the liposome and/or coupled to the steric stabilizer and/or coupled to the lipid bilayer.

6. Drug delivery system as claimed in any of the claims 1-5, wherein the compound exerts the said effect at the level of any of the components of the fibrinolytic pathway, the tissue factor and contact activation pathways (secondary hemostasis), or platelet function (primary hemostasis).

7. Drug delivery system as claimed in claim 6, wherein the compound is tranexamic acid (TA), e-aminocaproic acid (ACA), p-aminomethylbenzoic acid (AMBA), 4-aminomethyl-bicyclo-2,2,2-octane carboxylic acid (AMBOCA).

8. Drug delivery system as claimed in any one of the claims 6 and 7, comprising an agent that induces a procoagulant response by acting on components from the tissue factor and contact activation pathways.

9. Drug delivery system as claimed in any one of the claims 1-8, wherein the drug delivery platform comprises liposomes that further comprise a photosensitizer, in particular a phtalocyanine.

10. Drug delivery system as claimed in any one of the claims 1-9, wherein the drug delivery platform is provided with targeting molecules.

11. Drug delivery system as claimed in any one of the claims 1-10, wherein the drug delivery platform is formed by liposomes and the head group of the lipid is selected from the group consisting of: phosphatidylcholine, phosphocholine, phosphatidylethanolamine, phosphatidic acid, phosphatidylglycerol, phosphatidylserine, phosphatidylethanolamine, phosphatidylinositol, sphingomyelin, diglycerophosphate, glycerol, ethylene glycol, galloylglycerol and glycero-3-succinate.

12. Drug delivery system as claimed in any one of the claims 1-11, wherein the drug delivery platform comprises liposomes and at least part of the phospholipids that constitute the liposomes are dipalmitoyl phosphatidyl glycerol (DPPG) or dipalmitoyl phosphatidyl serine (DPPS).

13. Drug delivery system as claimed in any one of the claims 1-12, wherein the drug delivery platform comprises liposomes that are thermosensitive and wherein preferably at least part of the phospoholipids that constitute the liposomes are dipalmitoyl phosphatidylcholine (DPPC).

14. Use of a drug delivery system as claimed in any one of the claims 1-13 for the preparation of a medicament for the treatment of vascular and vessel-related pathologies.

15. Use as claimed in claim 14, wherein the pathology is a port wine stain.

Figure 1

CARBOXYFLUORESCEIN-LABELED PLATELETS

CARBOXYFLUORESCEIN-LABELED PLATELETS + HEPARIN

Figure 2

Figure 3

CONVENTIONAL PHOTOTHERMOLYSIS

SSPLT

Cross-sectional view

Red blood cell    Platelet    Drug carrier

Figure 4

**ANIONIC LIPOSOMES**

CONVENTIONAL LIPOSOMES

STEALTH LIPOSOMES

TARGETED LIPOSOMES

4. $Ca^{2+}$
$Ca^{2+}$ $Ca^{2+}$

Legend:

- Hydrophilic drug
- Hydrophobic drug
- Functionalized photosensitizer
- Hydrophobic anchor molecule
- Photocleavable group
- Antibody
- Antibody fragment
- Peptide

Figure 5

PARENT STRUCTURES

SUBSTITUTED
DERIVATIVES

PHTHALOCYANINE ($C_{32}H_{18}N_8$)

PHTHALOCYANINE

NAPHTHALOCYANINE ($C_{48}H_{26}N_8$)

NAPHTHALOCYANINE

CHLORINS ($C_{20}H_{16}N_4$)

CHLORINS

BACTERIOCHLORINS ($C_{20}H_{18}N_4$)

BACTERIOCHLORINS

Figure 6

Figure 7

Figure 8

1. DPPC:DSPE-PEG (98:2), 318 mM TA, n=2
2. DPPC:DSPE-PEG (96:4), 318 mM TA, n=6
3. DPPC:DSPE-PEG (94:6), 318 mM TA, n=9
4. DPPC:MPPC (90:10), 318 mM TA, n=3
5. DPPC:MPPC:DSPE-PEG (84:10:6), 318 mM TA, n=10

Figure 9

Figure 10

1. DPPC:DSPE-PEG (98:2), 318 mM TA, n=2
2. DPPC:DSPE-PEG (96:4), 318 mM TA, n=3
3. DPPC:DSPE-PEG (94:6), 318 mM TA, n=10
4. DPPC:MPPC (90:10), 318 mM TA, n=4
5. DPPC:MPPC:DSPE-PEG (84:10:6), 318 mM TA, n=6

Figure 11

Figure 12

# EUROPEAN SEARCH REPORT

Application Number

EP 09 15 1332

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HUERTAS-PEREZ ET AL: "Simple, rapid, and sensitive liquid chromatography-fluorescence method for the quantification of tranexamic acid in blood" JOURNAL OF CHROMATOGRAPHY, ELSEVIER SCIENCE PUBLISHERS B.V. AMSTERDAM, NL, vol. 1157, no. 1-2, 26 June 2007 (2007-06-26), pages 142-150, XP022131706 ISSN: 0021-9673 * page 145, left-hand column, line 1 - page 145, right-hand column, line 31 * ----- | 1-8, 11-15 | INV. A61K9/127 A61K31/198 A61K47/48 |
| X | US 5 505 954 A (WILLIAMS PATRICIA B [US] ET AL) 9 April 1996 (1996-04-09) * column 3, line 20 - column 5, line 22 * * claim 1 * ----- | 1-3,5-8, 11-15 | |
| X | US 5 547 680 A (WILLIAMS PATRICIA B [US] ET AL) 20 August 1996 (1996-08-20) * column 3, line 30 - column 3, line 40 * * column 5, line 1 - column 5, line 15 * * claim 1 * ----- | 1-3,5-8, 11-15 | TECHNICAL FIELDS SEARCHED (IPC) A61K |
| X | MANOSROI-A. PODJANASOONTHON-K. MANOSROI-J.: "Development of novel topical tranexamic acid liposome formulations" INTERNATIONAL JOURNAL OF PHARMACEUTICS, vol. 235, 2002, pages 61-70, XP002529293 * abstract * * table 1 * * page 62, right-hand column, line 27 - page 66, right-hand column, line 29 * ----- | 1-3,5-8, 11-15 | |

The present search report has been drawn up for all claims

| Place of search | Date of completion of the search | Examiner |
|---|---|---|
| Munich | 27 May 2009 | Schifferer, Hermann |

**ANNEX TO THE EUROPEAN SEARCH REPORT
ON EUROPEAN PATENT APPLICATION NO.**

EP 09 15 1332

This annex lists the patent family members relating to the patent documents cited in the above-mentioned European search report.
The members are as contained in the European Patent Office EDP file on
The European Patent Office is in no way liable for these particulars which are merely given for the purpose of information.

27-05-2009

| Patent document cited in search report | | Publication date | Patent family member(s) | Publication date |
|---|---|---|---|---|
| US 5505954 | A | 09-04-1996 | NONE | |
| US 5547680 | A | 20-08-1996 | NONE | |

EPO FORM P0459

For more details about this annex : see Official Journal of the European Patent Office, No. 12/82

REFERENCES CITED IN THE DESCRIPTION

**Patent documents cited in the description**

- US 6159938 A

**Non-patent literature cited in the description**

- **Hohenleutner U et al.** *J Invest Dermatol.,* May 1995, vol. 104 (5), 798-802
- **Fiskerstrand EJ et al.** *J Invest Dermatol.,* November 1996, vol. 107 (5), 671-5
- **Heger M et al.** *Opt Express.,* February 2005, vol. 13 (3), 702-15
- **Suthamjariya K et al.** *J Invest Dermatol.,* February 2004, vol. 122 (2), 518-25
- **Bezemer R et al.** *Opt Express.,* July 2007, vol. 15 (14), 8493-506
- **Greve B et al.** *Lasers Surg Med.,* 2004, vol. 34 (2), 168-73
- **Black JF et al.** *Photochem Photobiol.,* July 2004, vol. 80, 89-97
- **Tan OT et al.** *Arch Dermatol.,* September 1986, vol. 122 (9), 1015-22
- **Herczenik E et al.** *Arterioscler Thromb Vasc Biol.,* July 2007, vol. 27 (7), 1657-65
- **Maas C et al.** *J Clin Invest.,* September 2008, vol. 118 (9), 3208-18
- **Awasthi VD et al.** *Int J Pharm.,* 06 March 2003, vol. 253 (1-2), 121-32
- **Liu D et al.** *Biochim Biophys Acta,* 17 February 1992, vol. 1104 (1), 95-101
- **Klibanov AL et al.** *FEBS Lett.,* 30 July 1990, vol. 268 (1), 235-7
- **Senior J et al.** *Biochim Biophys Acta,* 11 February 1991, vol. 1062 (1), 77-82
- **Allen TM et al.** *Biochim Biophys Acta,* 01 July 1991, vol. 1066 (1), 29-36
- **Blume G et al.** *Biochim Biophys Acta,* 02 November 1990, vol. 1029 (1), 91-7
- **Torchilin VP et al.** *J Pharm Sci.,* September 1995, vol. 84 (9), 1049-53
- **Chiu GN et al.** *Biochim Biophys Acta,* 18 February 2002, vol. 1560 (1-2), 37-50
- **Chiu GN et al.** *Biochim Biophys Acta,* 27 June 2003, vol. 1613 (1-2), 115-21
- **Thompson DH et al.** *Methods Enzymol.,* 2004, vol. 387, 153-68
- **Mikus P ; Ny T.** Intracellular polymerization of the serpin plasminogen activator inhibitor type 2. *J Biol Chem.,* 26 April 1996, vol. 271 (17), 10048-53
- **Jones ME et al.** *Thromb Res.,* 15 September 1985, vol. 39 (6), 711-24
- **Klein S.** *Arterioscler Thromb Vasc Biol.,* October 2001, vol. 21 (10), 1695-700
- **Hu H et al.** *Thromb Res.,* 2005, vol. 116 (3), 241-71
- **Rui YJ et al.** *J Organic Chem.,* 1994, vol. 59 (19), 5758-5762
- **Shin J et al.** *J Org Chem.,* 22 August 2003, vol. 68 (17), 6760-6
- **Bondurant ; O'Brien.** *J. Am. Chem. Sec.,* 1998, vol. 120, 13541-13542
- **Bondurant B et al.** *Biochim Biophys Acta,* 09 March 2001, vol. 1511 (1), 113-22
- **Spratt T et al.** *Biochim Biophys Acta,* 01 April 2003, vol. 1611 (1-2), 35-43
- **Mueller A et al.** *Macromolecules,* 27 June 2000, vol. 33 (13), 4799-4804
- **Miller CR et al.** *FEBS Lett.,* 04 February 2000, vol. 467 (1), 52-6
- **Ricchelli F et al.** *Biochim Biophys Acta,* 30 December 1994, vol. 1196 (2), 165-71
- **de Oliveira CA et al.** *Chem Phys Lipids,* January 2005, vol. 133 (1), 69-78
- **Vittar NB et al.** *Int J Biochem Cell Biol.,* 2008, vol. 40 (10), 2192-205
- **Derycke AS et al.** *J Natl Cancer Inst.,* 03 November 2004, vol. 96 (21), 1620-30
- **Shin J et al.** *J Control Release,* 28 August 2003, vol. 91 (1-2), 187-200
- **Udenfriend S et al.** *Science,* 1972, vol. 178 (63), 871-872
- **Rouser G et al.** *Lipids,* 1970, vol. 5 (5), 494-6
- Liposomes. Oxford University Press, 2003, 64
- **Nagle JF et al.** *Curr. Opin. Struc. Biol.,* 2000, vol. 10 (4), 474-480
- **Majewski J et al.** *J. Am. Chem. Soc.,* 1998, vol. 120 (7), 1469-1473
- **Chi LM et al.** *Biophys. J.,* 1990, vol. 57 (6), 1225-1232
- **Tahara Y et al.** *Micron,* 1994, vol. 25 (2), 141-149
- **Nunes SM et al.** *Braz J Med Biol Res.,* February 2004, vol. 37 (2), 273-84

- **Hadjur C et al.** *Journal of Photochemistry and Photobiology B: Biology,* 1997, vol. 38, 196-202
- **Thompson DH et al.** *Biochim Biophys Acta,* 21 February 1996, vol. 1279 (1), 25-34
- **Bezemer R et al.** *Opt Express,* 25 July 2007, vol. 15 (4), 8493-8506
- Liposomes. Oxford University Press, 2003, 231-263
- **Heger M et al.** *Anal Quant Cytol Histol.*